# EUROPEAN PATENT SPECIFICATION

(11) **EP 2 742 940 B1**
(45) Date of publication and mention of the grant of the patent: **26.07.2017**
(21) Application number: 13195316.8
(22) Date of filing: 02.12.2013
(51) Int. Cl.: A61K 31/439, A61K 9/20, C07D 453/02, A61P 25/16, A61P 25/18, A61P 25/22, A61P 25/24, A61P 25/28

(54) **Fumarate salt of (R)-3-(6-(4-methylphenyl)-pyridin-3-yloxy)-l-aza-bicyclo-[2.2.2]octane for adminstration once daily, twice daily or thrice daily**
(R)-3-(6-(4-methylphenyl)-pyridin-3-yloxy)-l-aza-bicyclo-[2.2.2]octane Fumarat zur einmal, zweimal oder dreimal täglichen Verabreichung
Sel de fumarate de (R)-3-(6-(4-methylphenyl)-pyridin-3-yloxy)-l-aza-bicyclo-[2.2.2]octane destinés à être administrés une fois par jour, deux fois par jour ou trois fois par jour

(30) Priority: 13.12.2012 EP 12197100; 13.12.2012 EP 12197096; 20.12.2012 EP 12198771; 03.01.2013 EP 13150179; 10.01.2013 EP 13150891; 24.01.2013 EP 13152607
(43) Date of publication of application: 18.06.2014
(73) Proprietor: IP Gesellschaft für Management mbH, 50931 Köln (DE)
(72) Inventor: Trinius, Frank, 50931 Köln (DE)

(56) References cited:
- WO-A1-2004/022556
- WO-A1-2013/010679
- WO-A1-2013/010916

## Description

### BACKGROUND ART

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PSet al., Int J Infect Dis. 2002 Jun;6(2):108-12).

In Germany and other European countries, numerous pharmaceuticals are marketed in packaging wherein every single administration unit is individually packaged in a single packaging. Examples include but are not limited to Frubiase^{®} Calcium (Boehringer Ingelheim) marketed as a liquid administration unit that is individually packaged in a glass ampoule; Orthomol Immun^{®} Direktgranulat (Orthomol) marketed as administration unit in granulate form that is individually packaged in a bag; Orthomol Vital m^{®} (Orthomol) marketed as a liquid administration unit that is individually packaged in a bottle having a lid containing an individually packaged tablet; Vitasprint^{®} (Pfizer) and Celestamine^{®} (MSD Sharp & Dohme) each marketed as a liquid administration unit that is individually packaged in a bottle; Alka-Seltzer^{®} (Bayer) marketed as effervescent tablet that is individually packaged in a bag; Aspirin^{®} Complex (Bayer), Aspirin^{®} Effect (Bayer), Helmex^{®} (Infectopharm), Monuril^{®} Granulat (Pierre Fabre Pharma) each marketed as administration unit in granulate form that individually packaged in a bag; ellaOne^{®} (HRA Pharma), Levonelle^{®} (Bayer Schering Pharma); Pidana^{®} (HRA Pharma), Fungata^{®} (Pfizer), and Canesten^{®} Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging.

These objects have been achieved by the present invention.

### SUMMARY OF THE INVENTION

The present invention relates to a blister packaging comprising an administration unit, wherein the administration unit comprises a fumarate salt of (R)-3-(6-(4-methylphenyl)-pyridin-3-yloxy)-1-aza-bicyclo[2.2.2]octane; and wherein the administration unit is for use in the treatment of a disorder or a disease in a human, wherein the administration unit is administered once daily, or twice daily, or thrice daily.The invention concerns the fumarate salt of (R)-3-(6-(4-methylphenyl)-pyridin-3-yloxy)-1-aza-bicyclo[2.2.2]octane, and relates to a blister packaging comprising administration units comprising fumarate salt of (R)-3-(6-(4-methylphenyl)-pyridin-3-yloxy)-1-aza-bicyclo[2.2.2]octane.

The invention relates to a packaging comprising an administration units comprising a solid form of (R)-3-(6-(4-methylphenyl)-pyridin-3-yloxy)-1-aza-bicyclo[2.2.2]octane, namely to fumarate salt of (R)-3-(6-(4-methylphenyl)-pyridin-3-yloxy)-1-aza-bicyclo[2.2.2]octane, which is useful for treating prevention, treatment of a disease, in which nAChR ct7 activation plays a role, revention, treatment of of psychiatric or neurodegenerative disorders, CNS, PNS diseases, and inflammation.

The fumarate, maleate, chloride, phosphate, succinate or malonate salt of (R)-3-(6-(4-methylphenyl)-pyridin-3-yloxy)-1-aza-bicyclo[2.2.2]octane is described in WO2013010916, which is identical to WO2013010679. WO2013010916 contains the following information concerning the fumarate, maleate, chloride, phosphate, succinate and malonate salt of (R)-3-(6-(4-methylphenyl)-pyridin-3 -yloxy) -1-aza-bicyclo [2.2.2] octane:
1. Fumarate salt: In embodiment 1, the salt of the invention is the fumarate salt of (R)-3-(6-(4-methylphenyl)-pyridin-3-yloxy)-1-aza-bicyclo[2.2.2]octane, e.g. the mono-fumarate salt of (R)-3-(6-(4-methylphenyl)-pyridin-3-yloxy)-1-aza-bicyclo[2.2.2]octane in crystalline form. The mono-fumarate salt of (R)-3-(6-(4-methylphenyl)-pyridin-3-yloxy)-1-aza-bicyclo[2.2.2]octane in crystalline form may be produced from isopropyl alcohol when one equivalent fumaric acid is used.

The molecular formula is C₂₃H₂₆N₂O₅. It shows good solubility in aqueous media (>30mg/ml in water, 0.1 N Hcl, and pH 6.8 buffer). It is slightly hygroscopic: Loss on drying (LOD) of a sample was <0.03% and moisture gain was 0.5% at 85% relative humidity (RH). Its melting point was determined by heating at 2°C/minute to be 164-168.5°C (onset) with subsequent decomposition. It shows good stability in many buffer solutions and at various pH values. Also the solid-state stability is good. The X-ray powder diffraction (XRPD) pattern of a sample prepared according to this method (see also Example 1 of WO2013010916) is shown in Figure 1 of WO2013010916. Measurements were performed at a temperature of about 22°C and an x-ray wavelength, λ, of 1.5418A (CuKa λ=1.5418A). Summary of XRPD pattern:

| No. | 2 theta (deg°) | Intensity | | No. | 2 theta (deg°) | Intensity |
|---|---|---|---|---|---|---|
| 1 | 17.4 | 75.2 | | 13 | 29.3 | 18.5 |
| 2 | 15.2 | 49.8 | | 14 | 20.9 | 16.7 |
| 3 | 3.8 | 46.3 | | 15 | 26.5 | 15.8 |
| 4 | 20.1 | 45.2 | | 16 | 21.8 | 15.7 |
| 5 | 19.8 | 35.7 | | 17 | 30.8 | 9.1 |
| 6 | 13.7 | 33.8 | | 18 | 27.5 | 8.4 |
| 7 | 22.8 | 31.2 | | 19 | 7.6 | 8.3 |
| 8 | 19.2 | 25.9 | | 20 | 25.1 | 8 |
| 9 | 26.7 | 24 | | 21 | 23.2 | 7.6 |
| 10 | 18.5 | 22.5 | | 22 | 36.4 | 7.6 |
| 11 | 25.9 | 22.2 | | 23 | 23.9 | 7.4 |
| 12 | 11.3 | 21.5 | | 24 | 38.9 | 6.5 |

In one embodiment, the mono-fumarate salt of (R)-3-(6-(4-methylphenyl)-pyridin-3-yloxy)-1-aza-bicyclo[2.2.2]octane in crystalline form is characterized by an XRPD pattern with at least four, more preferably five, most preferably all of the folowing peaks at an angle of refraction 2 theta (20) of 3.8, 13.7, 15.2, 17.4, 19.8 and 20.1, ±0.2, respectively. In one embodiment, the mono-fumarate salt of (R)-3-(6-(4-methylphenyl)-pyridin-3-yloxy)-1-aza-bicyclo[2.2.2]octane in crystalline form is characterized by an XRPD pattern substantially the same as the XRPD pattern shown in Figure 1 of WO2013010916. The term "substantially the same" with reference to X-ray diffraction peak positions means that typical peak position and intensity variability are taken into account. For example, one skilled in the art will appreciate that the peak positions (20) will show some inter-apparatus variability, typically as much as 0.2°. Further, one skilled in the art will appreciate that peak intensities willshow inter-apparatus variability as well as variability due to degree of crystallinity, preferred orientation, prepared sample surface, and other factors known to those skilled in the art, and should be taken as qualitative measure only.

2. Maleate salt [Reference]: In comparative embodiment 2, the salt is the maleate salt of (R)-3-(6-(4-methylphenyl)-pyridin-3-yloxy)-1-aza-bicyclo[2.2.2]octane, e.g. the mono-maleate salt of (R)-3-(6-(4-methylphenyl)-pyridin-3-yloxy)-1-aza-bicyclo[2.2.2]octane in crystalline form. The mono-maleate salt of (R)-3-(6-(4-methylphenyl)-pyridin-3-yloxy)-1-aza-bicyclo[2.2.2]octane in crystalline form may be produced from acetonitrile when one equivalent maleic acid is used.

It shows good solubility in aqueous media (>30mg/ml in water, 0. IN HCl, and pH 6.8 buffer). It is slightly hygroscopic: LOD of a sample was <0.03% and moisture gain was 0.3% at 85% RH. Its melting point was determined by heating at 2°C/minute to be 152-154°C (onset) with subsequent decomposition. It shows good stability in many buffer solutions and at various pH values. Also the solid-state stability is good. The XRPD pattern of a sample prepared according to this method (see also Example 2) is shown in Figure 2 of WO2013010916. Measurements were performed at a temperature of about 22°C and an x-ray wavelength, λ, of 1.5418A (CuKa λ= 1.5 18A). Summary of XRPD pattern:

| No. | 2 theta (deg°) | Intensity | | No. | 2 theta (deg°) | Intensity |
|---|---|---|---|---|---|---|
| 1 | 19.1 | 199.1 | | 14 | 25.5 | 23.3 |
| 2 | 23.5 | 95.4 | | 15 | 27.9 | 21.7 |
| 3 | 18 | 80.1 | | 16 | 24.6 | 21.6 |
| 4 | 16 | 54.2 | | 17 | 18.6 | 17.8 |
| 5 | 12.9 | 46.3 | | 18 | 35 | 17.6 |
| 6 | 19.9 | 45.4 | | 19 | 21.9 | 15.9 |
| 7 | 12.6 | 45 | | 20 | 26 | 15.9 |
| 8 | 16.5 | 34.1 | | 21 | 26.4 | 15.6 |
| 9 | 30.5 | 32.1 | | 22 | 15.6 | 15.2 |
| 10 | 28.7 | 26 | | 23 | 29.8 | 14.4 |
| 11 | 24.9 | 25.6 | | 24 | 35.8 | 12 |
| 12 | 31.3 | 25.4 | | 25 | 33.2 | 11.7 |
| 13 | 9.5 | 25.1 | | 26 | 29.6 | 11.5 |

In one embodiment, the mono-maleate salt of (R)-3-(6-(4-methylphenyl)-pyridin-3-yloxy)-1-aza-bicyclo[2.2.2]octane in crystalline form is characterized by an XRPD pattern with at least four, more preferably five, most preferably all of the following peaks at an angle of refraction 2 theta (2Θ) of 12.9, 16.0, 18.0, 19.1 , 19.9 and 23.5, ±0.2, respectively. In one embodiment, the mono-maleate salt of (R)-3-(6-(4-methylphenyl)-pyridin-3-yloxy)-1-aza-bicyclo[2.2.2]octane in crystalline form is characterized by an XRPD pattern substantially the same as the XRPD pattern shown in Figure 2 of WO2013010916.

3. Chloride salt [Reference]: In comparative embodiment 3, the salt is the hydrochloride salt of (R)-3-(6-(4-methylphenyl)-pyridin-3-yloxy)-1-aza-bicyclo[2.2.2]octane, e.g. the mono-hydrochloride salt of (R)-3-(6-(4-methylphenyl)-pyridin-3-yloxy)-1-aza-bicyclo[2.2.2]octane in crystalline form. The mono-hydrochloride salt of (R)-3-(6-(4-methylphenyl)-pyridin-3-yloxy)-1-aza-bicyclo[2.2.2]octane in crystalline form may be produced directly from a synthesis mixture by adding hydrochloric acid. It shows good solubility in aqueous media (>30mg/ml). It is hygroscopic and may form mono- and/or di-hydrates depending on humidity level; when tested, LOD of a sample was about 5% and this amount of water was retained under normal conditions (i.e. at about 40-50% RH). Theoretically, an amout of 5.2% water correlates to 1 water molecule per salt molecule. Moisture gain was about 5% at 85% RH - which would correlate with 2 water molecules per salt molecule.

Its melting point was determined by heating at 2°C/minute to be 240°C (onset) with subsequent decomposition. The XRPD pattern of a sample prepared directly from a synthesis mixture (e.g. see Example 3.1.) is shown in Figure 3 of WO2013010916. Measurements were performed at a temperature of about 22°C and an xray wavelength, λ, of 1.5418A (CuKa λ=1.5418A). Summary of XRPD pattern:

| No. | 2 theta (deg°) | intensity | | No. | 2 theta (deg°) | Intensity |
|---|---|---|---|---|---|---|
| 1 | 20.8 | 125.1 | | 9 | 14.6 | 20.7 |
| 2 | 7.3 | 81.4 | | 10 | 23.5 | 19.9 |
| 3 | 17.2 | 50.9 | | 11 | 16.3 | 16.6 |
| 4 | 11.6 | 46.6 | | 12 | 28.8 | 12 |
| 5 | 18.4 | 46.3 | | 13 | 27.2 | 11.4 |
| 6 | 31.1 | 34.8 | | 14 | 25 | 10.9 |
| 7 | 26.7 | 34.3 | | 15 | 22.9 | 10.9 |
| 8 | 19.7 | 22.7 | | 16 | 13.5 | 8.9 |

In one embodiment, the mono-hydrochloride salt of (R)-3-(6-(4-methylphenyl)-pyridin-3-yloxy)-1-aza-bicyclo[2.2.2]octane in crystalline form is characterized by an XRPD pattern with at least four, more preferably five, most preferably all of the folowing peaks at an angle of refraction 2 theta (20) of 7.3, 11.6, 17.2, 18.4, 20.8 and 31.1, ±0.2, respectively. In one embodiment, the mono-hydrochloride salt of (R)-3-(6-(4-methylphenyl)-pyridin-3-yloxy)-1-aza-bicyclo[2.2.2]octane in crystalline form is characterized by an XRPD pattern substantially the same as the XRPD pattern shown in Figure 3 of WO2013010916.

4. Phosphate salt [Reference]: In comparative embodiment 4, the salt is the phosphate salt of (R)-3-(6-(4-methylphenyl)-pyridin-3-yloxy)-1-aza-bicyclo[2.2.2]octane, e.g. the mono-phosphate salt of (R)-3-(6-(4-methylphenyl)-pyridin-3-yloxy)-1-aza-bicyclo[2.2.2]octane in crystalline form. It was discovered, that said phosphate salt exists in more than one solid form.

### 4.1. Form A of the mono-phosphate salt:

A mono-phosphate salt of (R)-3-(6-(4-methylphenyl)-pyridin-3-yloxy)-1-aza-bicyclo[2.2.2]octane in crystalline form may be produced from ethanol when one equivalent phosphoric acid is used. The XRPD pattern of a sample prepared according to this method (see also Example 4.1 of WO2013010916) is shown in Figure 4A of WO2013010916. Measurements were performed at a temperature of about 22°C and an x-ray wavelength, λ, of 1.5418A (CuKa λ=1.5418A).cSummary of XRPD pattern:

| No. | 2 theta (deg°) | intensity | | No. | 2 theta (deg°) | Intensity |
|---|---|---|---|---|---|---|
| 1 | 17.7 | 76.3 | | 10 | 18.8 | 20.7 |
| 2 | 14.3 | 76 | | 11 | 12.2 | 16.3 |
| 3 | 18.2 | 75.1 | | 12 | 30.4 | 13.8 |
| 4 | 19.7 | 64.9 | | 13 | 25.7 | 13.2 |
| 5 | 16.5 | 56.3 | | 14 | 22.8 | 12.8 |
| 6 | 4.7 | 46.6 | | 15 | 22.5 | 12.6 |
| 7 | 20 | 40.2 | | 16 | 29.4 | 12.5 |
| 8 | 21.8 | 29.7 | | 17 | 35 | 10.6 |
| 9 | 26 | 24.4 | | | | |

In one embodiment, the mono-phosphate salt of (R)-3-(6-(4-methylphenyl)-pyridin-3-yloxy)- 1-aza-bicyclo[2.2.2]octane in crystalline form is characterized by an XRPD pattern with at least four, more preferably five, most preferably all of the folowing peaks at an angle of refraction 2 theta (20) of 4.7, 14.3, 16.5, 17.7, 18.2 and 19.7, ±0.2, respectively. In one embodiment, the mono-phosphate salt of (R)-3-(6-(4-methylphenyl)-pyridin-3-yloxy)-1-aza-bicyclo[2.2.2]octane in crystalline form is characterized by an XRPD pattern substantially the same as the XRPD pattern shown in Figure 4A of WO2013010916. Form A of the mono-phosphate salt shows good solubility in aqueous media (>30mg/ml). It is slightly hygroscopic: when tested, LOD of a sample was about 0.5% and moisture gain was 0.2% at 85% RH. Its melting/decomposition point was determined by heating at 2°C/minute to be about 222°C.

### 4.2. Form B of the phosphate salt:

Another form of the phosphate salt of (R)-3-(6-(4-methylphenyl)-pyridin-3-yloxy)-1-aza-bicyclo[2.2.2]octane in crystalline form (Form B of the phosphate salt) was found as described in the Examples section (see Example 4.2 of WO2013010916). The associated XRPD pattern is shown in Figure 4B of WO2013010916.

### Summary of XRPD pattern:

| No. | 2 theta (deg°) | Intensity | | No. | 2 theta (deg°) | Intensity |
|---|---|---|---|---|---|---|
| 1 | 14.5 | 131.9 | | 21 | 20.7 | 13.6 |
| 2 | 14 | 106.9 | | 22 | 24.7 | 12.9 |
| 3 | 14.2 | 101.1 | | 23 | 21 | 12.7 |
| 4 | 15.6 | 62.4 | | 24 | 23.5 | 12.5 |
| 5 | 19.7 | 51.5 | | 25 | 25.8 | 11.2 |
| 6 | 19.3 | 42.3 | | 26 | 9 | 11.1 |
| 7 | 13.1 | 36 | | 27 | 9.6 | 10.8 |
| 8 | 16.8 | 34.2 | | 28 | 30.5 | 9.6 |
| 9 | 20 | 32.6 | | 29 | 25.2 | 9.4 |
| 10 | 18 | 30.4 | | 30 | 33.8 | 9.3 |
| 11 | 12.4 | 29.2 | | 31 | 27.1 | 9.1 |
| 12 | 22.6 | 24.4 | | 32 | 33.3 | 8.8 |
| 13 | 16.4 | 20.7 | | 33 | 30.1 | 8 |
| 14 | 4.2 | 19.3 | | 34 | 7.8 | 8 |
| 15 | 10.4 | 18 | | 35 | 31.5 | 7.5 |
| 16 | 23 | 17.8 | | 36 | 8.3 | 7.2 |
| 17 | 11.5 | 17.4 | | 37 | 29.6 | 7.2 |
| 18 | 15.1 | 15.6 | | 38 | 26.7 | 6.8 |
| 19 | 32.6 | 14.2 | | 39 | 29.1 | 6.4 |
| 20 | 12 | 13.7 | | 40 | 7.1 | 6.2 |

### 4.3. Form C of the phosphate salt:

Another form of the phosphate salt of (R)-3-(6-(4-methylphenyl)-pyridin-3-yloxy)-1-aza-bicyclo[2.2.2]octane in crystalline form (Form C of the phosphate salt) was found as described in the Examples section (see Example 4.3 of WO2013010916). The associated XRPD pattern is shown in Figure 4C of WO2013010916. Summary of XRPD pattern:

| No. | 2 theta (deg°) | Intensity | | No. | 2 theta (deg°) | Intensity |
|---|---|---|---|---|---|---|
| 1 | 4.6 | 155.5 | | 9 | 20.2 | 36.2 |
| 2 | 14.9 | 68.8 | | 10 | 25.8 | 32.8 |
| 3 | 16.9 | 54.8 | | 11 | 18 | 22.9 |
| 4 | 17.7 | 50.4 | | 12 | 30.1 | 17.4 |
| 5 | 19.9 | 47.6 | | 13 | 22.7 | 16.2 |
| 6 | 18.6 | 44.6 | | 14 | 29.4 | 16 |
| 7 | 13.9 | 44.5 | | 15 | 12.1 | 11.1 |
| 8 | 21.6 | 37.5 | | 16 | 7.2 | 9.7 |

### 5. Succinate salt [Reference]:

In comparative embodiment 5, the salt is the succinate salt of (R)-3-(6-(4-methylphenyl)-pyridin-3-yloxy)-1-aza-bicyclo[2.2.2]octane, e.g. the mono-succinate salt of (R)-3-(6-(4-methylphenyl)-pyridin-3-yloxy)-1-aza-bicyclo[2.2.2]octane in crystalline form. It was discovered, that said mono-succinate salt exists in more than one solid form.

### 5.1 Form A of the mono-succinate salt

A mono-succinate salt of (R)-3-(6-(4-methylphenyl)-pyridin-3-yloxy)-1-aza-bicyclo[2.2.2]octane in crystalline form may be produced from ethanol when one equivalent succinic acid is used. The XRPD pattern of a sample prepared according to this method (see also Example 5 of WO2013010916) is shown in Figure 5A of WO2013010916. Measurements were performed at a temperature of about 22°C and an x-ray wavelength, λ, of 1.5418A (CuKa A=1.5418A). Summary of XRPD pattern:

| No. | 2 theta (deg°) | Intensity | | No. | 2 theta (deg°) | Intensity |
|---|---|---|---|---|---|---|
| 1 | 17.4 | 178.5 | | 11 | 14.8 | 17.1 |
| 2 | 19.4 | 161.1 | | 12 | 9.2 | 15.2 |
| 3 | 10.7 | 45.4 | | 13 | 29.1 | 15 |
| 4 | 15.2 | 38.7 | | 14 | 23 | 14.6 |
| 5 | 15.8 | 31.5 | | 15 | 6.5 | 14.6 |
| 6 | 23.7 | 28.2 | | 16 | 25.3 | 13.1 |
| 7 | 13.3 | 28.1 | | 17 | 30 | 12.5 |
| 8 | 21.8 | 24.8 | | 18 | 24.6 | 11.4 |
| 9 | 18.4 | 23.7 | | 19 | 32.9 | 11 |
| 10 | 12.9 | 19.5 | | | | |

In one embodiment, the mono-succinate salt of (R)-3-(6-(4-methylphenyl)-pyridin-3-yloxy)-1-aza-bicyclo[2.2.2]octane in crystalline form is characterized by an XRPD pattern with at least four, more preferably five, most preferably all of the folowing peaks at an angle of refraction 2 theta (20) of 10.7, 15.2, 15.8, 17.4, 19.4 and 23.7, ±0.2, respectively. In one embodiment, the mono-succinate salt of (R)-3-(6-(4-methylphenyl)-pyridin-3-yloxy)-1-aza-bicyclo[2.2.2]octane in crystalline form is characterized by an XRPD pattern substantially the same as the XRPD pattern shown in Figure 5A of WO2013010916. Form A of the the mono-succinate salt shows good solubility in aqueous media (2-15mg/ml). It is considered to be a mono-hydrate: when tested, LOD of a sample was about 4.5%. Theoretically, an amout of 4.1 % water correlates to 1 water molecule per salt molecule. Moisture gain was 0.3% at 85% RH. Its melting point was determined by heating at 2°C/minute to be 113°C (onset) with subsequent decomposition.

### 5.2. Form B of the mono-succinate salt [Reference]:

Another form of the succinate salt of (R)-3-(6-(4-methylphenyl)-pyridin-3-yloxy)-1-aza-bicyclo[2.2.2]octane in crystalline form (Form B of the mono-succinate salt) was found as described in the Examples section (see Example 5.2 of WO2013010916). The associated XRPD pattern is shown in Figure 5B of WO2013010916. Summary of XRPD pattern:

| No. | 2 theta (deg°) | Intensity | | No. | 2 theta (deg°) | Intensity |
|---|---|---|---|---|---|---|
| 1 | 7.7 | 2279 | | 11 | 16.9 | 462 |
| 2 | 18.1 | 1417 | | 12 | 25.5 | 423 |
| 3 | 3.8 | 909 | | 13 | 18.8 | 418 |
| 4 | 20.8 | 901 | | 14 | 21.9 | 367 |
| 5 | 17 | 816 | | 15 | 22.7 | 306 |
| 6 | 22.3 | 765 | | 16 | 27.4 | 301 |
| 7 | 15.5 | 639 | | 17 | 24.5 | 292 |
| 8 | 23.9 | 578 | | 18 | 13.8 | 273 |
| 9 | 24 | 564 | | 19 | 11.6 | 226 |
| 10 | 19.4 | 463 | | | | |

### 6. Malonate salt [Reference]:

In comparative embodiment 6, the salt is the malonate salt of (R)-3-(6-(4-methylphenyl)-pyridin-3-yloxy)-1-aza-bicyclo[2.2.2]octane, e.g. the mono-malonate salt of (R)-3-(6-(4-methylphenyl)-pyridin-3-yloxy)-1-aza-bicyclo[2.2.2]octane in crystalline form. The mono-malonate salt of (R)-3-(6-(4-methylphenyl)-pyridin-3-yloxy)-1-aza-bicyclo[2.2.2]octane in crystalline form may be produced from acetonitrile when one equivalent malonic acid is used. It shows good solubility in aqueous media (>30mg/ml). It is slightly hygroscopic: when tested, LOD of a sample was 0% and moisture gain was 1.3% at 85% RH. Its melting point was determined by heating at 2°C/minute to be 140°C (onset) with subsequent decomposition. The XRPD pattern of a sample prepared according to this method (see also Example 6 of WO2013010916) is shown in Figure 6 of WO2013010916. Measurements were performed at a temperature of about 22°C and an x-ray wavelength, λ, of 1.5418A (CuKa λ=1.5418A). Summary of XRPD pattern:

| No. | 2 theta (deg°) | Intensity | | No. | 2 theta (deg°) | Intensity |
|---|---|---|---|---|---|---|
| 1 | 5 | 78.1 | | 16 | 27 | 20.1 |
| 2 | 24.3 | 75 | | 17 | 20.9 | 18 |
| 3 | 16.8 | 63.2 | | 18 | 30.1 | 17.9 |
| 4 | 18.1 | 55.8 | | 19 | 32.2 | 14.3 |
| 5 | 13 | 53.2 | | 20 | 31 | 13.4 |
| 6 | 19.8 | 53 | | 21 | 14.3 | 13.3 |
| 7 | 17.1 | 52.3 | | 22 | 10.2 | 11.8 |
| 8 | 15.6 | 51.9 | | 23 | 21.4 | 11.3 |
| 9 | 20.2 | 50.7 | | 24 | 34 | 10.6 |
| 10 | 18.7 | 33 | | 25 | 33.5 | 10.5 |
| 11 | 15.1 | 31.3 | | 26 | 25.9 | 9.4 |
| 12 | 29.1 | 28.5 | | 27 | 25.2 | 9.1 |
| 13 | 12.2 | 23.6 | | 28 | 34.5 | 8.7 |
| 14 | 28.8 | 23.5 | | 29 | 35.8 | 8.3 |
| 15 | 27.5 | 21.4 | | 30 | 35.2 | 7.2 |

In one embodiment, the mono-malonate salt of (R)-3-(6-(4-methylphenyl)-pyridin-3-yloxy)-1-aza-bicyclo[2.2.2]octane in crystalline form is characterized by an XRPD pattern with at least four, more preferably five, most preferably all of the following peaks at an angle of refraction 2 theta (20) of 5.0, 13.0, 16.8, 18.1 , 19.8 and 24.3, ±0.2, respectively. In one embodiment, the mono-malonate salt of (R)-3-(6-(4-methylphenyl)-pyridin-3-yloxy)-1-aza-bicyclo[2.2.2]octane in crystalline form is characterized by an XRPD pattern substantially the same as the XRPD pattern shown in Figure 6 of WO2013010916. Preparation methods for crystalline forms: Crystalline forms may be prepared by a variety of methods, including for example, crystallization or recrystallization from a suitable solvent, sublimation, growth from a melt, solid state transformation from another phase, crystallization from a supercritical fluid, and jet spraying. Techniques for crystallization or recrystallization of crystalline forms from a solvent mixture include, for example, evaporation of the solvent, decreasing the temperature of the solvent mixture, crystal seeding a supersaturated solvent mixture of the molecule and/or salt, freeze drying the solvent mixture, and addition of antisolvents (countersolvents) to the solvent mixture. High throughput crystallization techniques may be employed to prepare crystalline forms including polymorphs. Crystals of drugs, including polymorphs, methods of preparation, and characterization of drug crystals are discussed in Solid-State Chemistry of Drugs, S.R. Byrn, R.R. Pfeiffer, and J.G. Stowell, 2nd Edition, SSCI, West Lafayette, Indiana (1999). For crystallization techniques that employ solvent, the choice of solvent or solvents is typically dependent upon one or more factors, such as solubility of the compound, crystallization technique, and vapor pressure of the solvent. Combinations of solvents may be employed, for example, the compound may be solubilized into a first solvent to afford a solution, followed by the addition of an antisolvent to decrease the solubility of the compound in the solution and to afford the formation of crystals. An antisolvent is a solvent in which the compound has low solubility. In one method to prepare crystals, a compound is suspended and/or stirred in a suitable solvent to afford a slurry, which may be heated to promote dissolution. The term "slurry", as used herein, means a saturated solution of the compound, which may also contain an additional amount of the compound to afford a heterogeneous mixture of the compound and a solvent at a given temperature. Seed crystals may be added to any crystallization mixture to promote crystallization (see "Programmed Cooling of Batch Crystallizers," J.W. Mullin and J. Nyvlt, Chemical Engineering Science, 1971, 26, 369-377). In general, seed crystals of small size are used. Seed crystals of small size may be generated by sieving, milling, or micronizing of large crystals, or by micro-crystallization of solutions. Care should be taken that milling or micronizing of crystals does not result in any change in crystallinity form the desired crystal form (i.e., change to amorphous or to another polymorph). A cooled crystallization mixture may be filtered under vacuum, and the isolated solids may be washed with a suitable solvent, such as cold recrystallization solvent, and dried under a nitrogen purge to afford the desired crystalline form. The isolated solids may be analyzed by a suitable spectroscopic or analytical technique, such as solid state nuclear magnetic resonance, differential scanning calorimetry, x-ray powder diffraction, or the like, to assure formation of the preferred crystalline form of the product. The resulting crystalline form is typically produced in an amount of greater than about 70 weight % isolated yield, preferably greater than 90 weight % isolated yield, based on the weight of the compound originally employed in the crystallization procedure. The product may be delumped by sieving or forced sieving, if necessary.

Crystalline forms may be prepared directly from the reaction medium of the final process for preparing (R)-3-(6-(4-methylphenyl)-pyridin-3-yloxy)-1-aza-bicyclo[2.2.2]octane or a salt of the invention. This may be achieved, for example, by employing in the final process step a solvent or a mixture of solvents from which the salt of the invention may be crystallized. Alternatively, crystalline forms may be obtained by distillation or solvent addition techniques. Suitable solvents for this purpose include, for example, nonpolar solvents and polar solvents, including protic polar solvents such as alcohols, and aprotic polar solvents such as ketones. The presence of more than one polymorph in a sample may be determined by techniques such as powder x-ray diffraction (PXRD) or solid state nuclear magnetic resonance spectroscopy. For example, the presence of extra peaks in the comparison of an experimentally measured PXRD pattern with a simulated PXRD pattern may indicate more than one polymorph in the sample. The simulated PXRD may be calculated from single crystal x-ray data; see Smith, D.K., UA FORTRAN Program for Calculating X-Ray Powder Diffraction Patterns," Lawrence Radiation Laboratory, Livermore, California, UCRL-7196 (April 1963). In many cooled and/or seeded crystallizations of the mono-fumarate salt of (R)-3-(6-(4-methylphenyl)-pyridin-3-yloxy)-1-aza-bicyclo[2.2.2]octane fine particles are obtained. Fine particles typically have the drawback of bad filtration properties and poor flowability, which is especially disadvantageous for the widely used dry-granulation using roller compaction. It was found that, depending on crystallization techniques, a mean particle size of the crystals of above 15 µητ 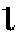 can be obtained. As laid out above, such mean particle sizes are especially suitable for formulation work. The term "mean particle size" (X5o) refers to a crystal size distribution wherein 50% of crystals related to the total volume of particles have a smaller diameter of an equivalent sphere than the value given. The term "X90" refers to a crystal size distribution wherein 90% of crystals related to the total volume of particles have a smaller diameter of an equivalent sphere than the value given. The term "X10" refers to a crystal size distribution wherein 10% of crystals related to the total volume of particles have a smaller diameter of an equivalent sphere than the value given.

Consequently, one embodiment of the invention is a method of preparing a mono-fumarate salt of (R)-3-(6-(4-methylphenyl)-pyridin-3-yloxy)-1-aza-bicyclo[2.2.2]octane in crystalline form comprising the steps of
(a) preparing a solution of a mono-fumarate salt of (R)-3-(6-(4-methylphenyl)-pyridin-3-yloxy)-1-aza-bicyclo[2.2.2]octane in a solvent mixture of a primary alcohol, a secondary alcohol and water, wherein the primary alcohol : secondary alcohol volume ratio is from 9 : 1 to 1 : 1 , and wherein the alcohols : water volume ratio is from 9 : 1 to 19 : 1;
(b) heating the solution of step (a) to elevated temperature;
(c) adding the solution of step (b) gradually to an ether antisolvent at a temperature ranging from ambient temperature to 55DC until a solution from step (b) : ether antisolvent volume ratio from 1 : 1 to 1 : 5 is reached; wherein after an amount of the solution of step (b) from 10% to 40% of the total amount is added, the resulting solution is seeded with seed crystals of a mono-fumarate salt of (R)-3-(6-(4-methylphenyl)-pyridin-3-yloxy)-1-aza-bicyclo[2.2.2]octane in crystalline form, wherein the seed crystals are suspended in a secondary alcohol;
(d) cooling the seeded solution of step (c) gradually to a temperature below ambient; and
(e) isolate the solids by filtration to obtain the mono-fumarate salt of (R)-3-(6-(4-methylphenyl)-pyridin-3-yloxy)-1-aza-bicyclo[2.2.2]octane in crystalline form.

Examples of primary alcohols are methanol or ethanol. An example of a secondary alcohol is isopropanol. An example of an ether antisolvent is tertiary-butylmethylether. Typically, the mean particle size of the seed crystals is from 1 µ 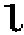 τ 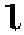 to 10 µητ Typically, the seed crystals are added in an amount of from 0.08% to 2% of the amount of the salt in step (a).

One embodiment of the invention is a method of preparing a mono-fumarate salt of (R)-3-(6-(4-methylphenyl)-pyridin-3-yloxy)-1-aza-bicyclo[2.2.2]octane in crystalline form comprising the steps of
(a) preparing a solution of a mono-fumarate salt of (R)-3-(6-(4-methylphenyl)-pyridin-3-yloxy)-1-aza-bicyclo[2.2.2]octane in a mixture of ethanol, isopropanol and water, wherein the ethanol : isopropanol volume ratio is about 75 : 15, and wherein the alcohols : water volume ratio is about 90 : 10;
(b) heating the solution of step (a) to a temperature of about 50°C;
(c) adding the solution of step (b) gradually to tertiary-butylmethylether at a temperature of about 50°C until a a solution from step (b) : tertiary-butylmethylether volume ratio of about 75 : 25 is reached; wherein after an amount of the solution of step (b) of about 25% of the total amount is added, the resulting solution is seeded with seed crystals of a mono-fumarate salt of (R)-3-(6-(4-methylphenyl)-pyridin-3-yloxy)-1-aza-bicyclo[2.2.2]octane in crystalline form, wherein the mean particle size of the seed crystals is about 10 µ 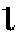 τ 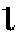 , wherein the seed crystals are added in an amount of about 0.08% of the amount of the salt in step (a), and wherein the seed crystals are suspended in isopropanol;
(d) cooling the seeded solution of step (c) gradually to a temperature ranging of about 0°C; and
(e) isolate the solids by filtration to obtain the mono-fumarate salt of (R)-3-(6-(4-methylphenyl)-pyridin-3-yloxy)-1-aza-bicyclo[2.2.2]octane in crystalline form.

One further embodiment of the invention is mono-fumarate salt of (R)-3-(6-(4-methylphenyl)-pyridin-3-yloxy)-1-aza-bicyclo[2.2.2]octane in crystalline form, wherein the mean particle size of the crystals is at least 15 µ 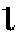 τ 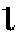 . One further embodiment of the invention is mono-fumarate salt of (R)-3-(6-(4-methylphenyl)-pyridin-3-yloxy)-1-aza-bicyclo[2.2.2]octane in crystalline form, wherein the mean particle size of the crystals is at least 20 pm. One further embodiment of the invention is mono-fumarate salt of (R)-3-(6-(4-methylphenyl)-pyridin-3-yloxy)-1-aza-bicyclo[2.2.2]octane in crystalline form, wherein the mean particle size of the crystals is at least 25 pm. One further embodiment of the invention is mono-fumarate salt of (R)-3-(6-(4-methylphenyl)-pyridin-3-yloxy)-1-aza-bicyclo[2.2.2]octane in crystalline form, wherein the mean particle size of the crystals is from 20 pm to 35 pm. One further embodiment of the invention is mono-fumarate salt of (R)-3-(6-(4-methylphenyl)-pyridin-3-yloxy)-1-aza-bicyclo[2.2.2]octane in crystalline form, wherein the mean particle size of the crystals is from 20 pm to 35 pm; the X10 is from 3 pm to 10 pm; and the X90 is from 70 pm to 90 pm. One further embodiment of the invention is mono-fumarate salt of (R)-3-(6-(4-methylphenyl)-pyridin-3-yloxy)-1-aza-bicyclo[2.2.2]octane in crystalline form, wherein the mean particle size of the crystals is from 25 pm to 30 pm.

Analysis of solid forms: The solid form of a salt of the invention may be characterized using various techniques, the operation of which are well known to those of ordinary skill in the art. The forms may be characterized and distinguished using single crystal x-ray diffraction, which is based on unit cell measurements of a single crystal of the form at a fixed analytical temperature. A detailed description of unit cells is provided in Stout & Jensen, X-Ray Structure Determination: A Practical Guide, Macmillan Co., New York (1968), Chapter 3. Alternatively, the unique arrangement of atoms in spatial relation within the crystalline lattice may be characterized according to the observed fractional atomic coordinates. Another means of characterizing the crystalline structure is by powder x-ray diffraction analysis in which the diffraction profile is compared to a simulated profile representing pure powder material, both run at the same analytical temperature, and measurements for the subject form characterized as a series of 20 values (usually four or more). Other means of characterizing the form may be used, such as solid state nuclear magnetic resonance (NMR), differential scanning calorimetry, thermography and gross examination of the crystalline or amorphous morphology. These parameters may also be used in combination to characterize the subject form. Mean particle sizes, X90 and X10 are typically measured by Fraunhofer light diffraction.

### Examples of WO2013010916:

The experimental section of WO2013010916 is summarized below:
Reference Example A1: Preparation/Characterization of free base of (R)-3-(6-(4- methylphenyl)-pyridin-3-yloxy)-1-aza-bicyclo[2.2.2]octane in crystalline form (Form A): About 8mg of (R)-3-(6-(4-methylphenyl)-pyridin-3-yloxy)-1-aza-bicyclo[2.2.2]octane in free base form dissolved in 0.2ml methanol was dried in vacuum at 40°C for >5hours. After drying, acetonitrile was added to the solid residue and the mixture was heated to 40°C and vortexed for about 2hours. The mixture was dried and the remaining solid was analyzed by XPRD. Following this method, a pattern as shown in Figure 7A of WO2013010916 (Form A) can be obtained. Form A of the free base shows low solubility in aqueous media (0.05mg/ml). It is hygroscopic: when tested, Loss on drying (LOD) of a sample was 0.1% and moisture gain was 2% at 93% relative humidity (RH). Its melting point was determined by heating at 2°C/minute to be 106°C (onset) with subsequent decomposition.
Reference Example A2: Preparation/Characterization of free base of (R)-3-(6-(4-methylphenyl)-pyridin-3-yloxy)-1-aza-bicyclo[2.2.21octane in crystalline form (Form B): About 8mg of (R)-3-(6-(4-methylphenyl)-pyridin-3-yloxy)-1-aza-bicyclo[2.2.2]octane in free base form dissolved in 0.2ml methanol was dried in vacuum at 40°C for >5hours. After drying, ethanol was added to the solid residue and the mixture was heated to 40°C and vortexed for about 2hours. The mixture was dried and the remaining solid was analyzed by XPRD (see Figure 7B of WO2013010916, Form B). The same experiment was performed using ethanol or isopropanol as solvent. Basically the same XPRD pattern was obtained. As all three solid forms gained an XPRD pattern as described under Reference Example A1 upon further drying, it was concluded that this new form is an alcohol solvate with low association temperature.

Example 1: Preparation of mono-fumarate salt of (R)-3-(6-(4-methylphenyl)-pyridin-3-yloxy)- 1-aza-bicyclof2.2.21octane in crystalline form: 500 mg of (R)-3-(6-(4-methylphenyl)-pyridin-3-yloxy)-1-aza-bicyclo[2.2.2]octane in free base form were suspended in 20 ml isopropyl alcohol. A stochtometric amount of fumaric acid was added. The resulting solution was stirred at ambient tempertature for 14 hours. The precipitate was collected by filtration and analyzed by proton-N R and XRPD (see Figure 1 of WO2013010916). Yield was 85%. Analysis of proton-NMR confirmed salt formation, a base/acid ratio of about 1:1 and the fact that the salt was not a solvate.

### Example 1.1: Preparation of mono-fumarate salt of (R)-3-(6-(4-methylphenyl)-pyridin-3- yloxy)-1-aza-bicyclo[2.2.21octane in crystalline form by seeded crystallization:

a) Preparation: 7.3 g mono-fumarate of (R)-3-(6-(4-methylphenyl)-pyridin-3-yloxy)-1-aza-bicyclo[2.2.2]octane (purity >98%; prepared as described e.g. in Example 13.2) was dissolved in ethanol (42.9g)/isopropanol (8.5g)/water (7.2g) at about 50°C, clarified by filtration and added at this temperature gradually over a period of about 8 hours to filtered tertiary-butylmethylether (118.4g) at a temperature of about 50°C. After about 25% of the filtrate was added, an ultrasonificated suspension of seed crystals of the mono-fumarate of (R)-3-(6-(4- methylphenyl)-pyridin-3-yloxy)-1-aza-bicyclo[2.2.2]octane (6mg, prepared e.g. as described in Example 13.2) in isopropanol (0.1 ml) was added to induce crystallization. The product suspension was maintained for another 1 hour at 50<e>C and cooled to 0°C within 8 hours. After another 1 hour at this temperature the solids were isolated by filtration, washed with isopropanol/tertiary-butylmethylether (40ml, 1:1 mixture) and dried at about 50°C under reduced pressure to yield the mono-fumarate of (R)-3-(6-(4-methylphenyl)-pyridtn-3-yloxy)-1- aza-bicyclo[2.2.2]octane (5.85 g; 81% of theory; purity >99.5%).
b) Characterization: Particle size measurements by Fraunhofer light diffraction
   Result: Mono-fumarate salt of (R)-3-(6-(4-methylphenyl)-pyridin-3-yloxy)-1-aza-bicyclo[2.2.2]octane in crystalline form prepared as described according to Example 1.1 was tested. The following values were obtained: x10 = 5.6+-0.5[mu][eta][iota]; x50 = 26.8+-1.3[mu][iota][eta] and xgo = 77.3+-3.3[mu][iota][eta] (N=8).
   Procedure: To about 0.5 g of test substance add some drops of the dispersing aid (1% Octastat 5000 (Octel Corp.) in white spirit (Sangajol, Schweizerhall Chemie)). Mix intensively on a vortex mixer, in order to wet the substance thoroughly and to form a smooth and homogeneous paste. Dilute the paste with white spirit to a final volume of 3 - 6 ml and mix the dispersion again. Determine the cumulative volume distribution using a laser diffraction instrument, e.g. determine the particle sizes at the undersize values of 10%, 50% and 90% ([Chi][iota]0, [Chi]50, X90). Measuring device: Sympatec HELOS (Sympatec GmbH; focal length: 500mm, optical concentration>= 5%, duration of measurement: 40 sec). Dispersion device: Suspension cell (QUIXEL, Sympatec GmbH).

Example 2: Preparation of mono-maleate salt of (R)-3-(6-(4-methylphenyl)-pyridin-3-yloxy)-1- aza-bicvclof2.2.21octane in crystalline form [reference]: 500 mg of (R)-3-(6-(4-methylphenyl)-pyridin-3-yloxy)-1-aza-bicyclo[2.2.2]octane in free base form were suspended in 5 ml acetonitrile. A stochiometric amount of maleic acid was added. The resulting solution was stirred at ambient tempertature for 14 hours. The precipitate was collected by filtration and analyzed by proton-NMR and XRPD (see Figure 2 of WO2013010916). Yield was 63%. Analysis of proton-NMR confirmed salt formation, a base/acid ratio of about 1: 1 and the fact that the salt was not a solvate.

Example 3: Preparation of mono-hydrochloride salt of (R)-3-(6-(4-methylphenvD-pyridin-3-yloxy)-1-aza-bicyclo-[2.2.2]octane in crystalline form [reference]: AIL reactor, equipped with a mechanical stirrer, digital thermometer, nitrogen inlet-outlet, reflux condenser and heating mantle was charged with 14.4g (R)-3-quinuclidinol, 176g (160mL) dimethylsulfoxide and 69.5g (78mL) 20wt% potassium tert-butoxide in tetrahydrofuran. The mixture was stirred at 23°C for 15 minutes and then heated to 95-110°C over a period of 1 hour to distill off about 40mL of tetrahydrofuran. Distilling was continued at 110°C for 30 minutes. The mixture was cooled to 90°C over a period of 20 minutes. Portionwise, 21 g 5-chloro-2-p-tolylpyridine was added. 1 1 g (20mL) dimethylsulfoxide was added. The reaction mixture was heated to 100°C over a period of 20 minutes and kept at said temperature for 3 hours. The mixture was cooled to 15°C over a period of 1 hour. 370g (500ml) tert-butyl methyl ether was added. 250g water was added over a period of 30 minutes, while maintaining the temperature below 25°C. The mixture was stirred for 30 minutes. The layers were separated and a solution of 102g 20%(v/v) aqueous sodium chloride was added to the organic layer. The mixture was stirred for 15 minutes and the layers were separated. The organic layer was filtered. A 1L reactor, equipped with a mechanical stirrer, digital thermometer, addition funnel, nitrogen inlet-outlet, reflux condenser and heating mantle was charged with the above organic layer. 109g (120mL) peroxide-free 2-propanol was added. A solution of 16.5g (17.8ml) 5.3 N HCl in 2-propanol was added over a period of 40 minutes. The mixture was heated to 53°C and stirred for 30 minutes. The mixture was cooled to 23°C over a period of 30 minutes and stirred for 1 hour. The solid was collected by filtration and washed with a solution of 2X 37g (50ml) of 1 %(v/v) peroxide-free 2-propanol / tert-butyl methyl ether. The solid was dried at 55°C under reduced pressure to afford 20.9g of the mono-hydrochloride salt of (R)-3-(6-(4-methylphenyl)-pyridin-3-yloxy)-1-aza-bicyclo[2.2.2]octane. The material was analyzed by XRPD (see Figure 3 of WO2013010916).

Example 4.1: Preparation of mono-phosphate salt of (R)-3-(6-(4-methylphenyl)-pyridin-3-yloxy[3/4]-1-aza-bicyclo-[2.2.2]octane in crystalline form (Form A) [reference]: 500 mg of (R)-3-(6-(4-methylphenyl)-pyridin-3-yloxy)-1-aza-bicyclo-[2.2.2]octane in free base form were suspended in 20 ml ethanol. A stochiometric amount of phosphoric acid was added. The resulting solution was stirred at ambient tempertature for 14 hours. The precipitate was collected by filtration and analyzed by proton-NMR and XRPD (see Figure 4A of WO2013010916, Form A). Yield was 77%. Analysis of proton-NMR confirmed salt formation and the fact that the salt was not a solvate. Elemental analysis was performed confirming a base/acid ratio of about 1:1 (C 57.9% (58.1%), H 6.7% (6.4%), N 7.1 % (7.1%) and P 7.7% (7.9%), theoretical values in brackets).

Example 4.2: Preparation of phosphate salt of (R)-3-(6-(4-methylphenyl)-pyridin-3-yloxy)-1- aza-bicyclo[2.2.21octane in crystalline form (Form B) [reference]: About 2.3mg of (R)-3-(6-(4-methylphenyl)-pyridin-3-yloxy)-1-aza-bicyclo[2.2.2]octane in free base form was dissolved in 0.15ml ethanol. A stochiometric amount of phosphoric acid was added. The mixture was dried in vacuum at 40°C for >5hours. After drying, 0.1ml ethanol and 0.05ml water were added. The mixture was heated to 40°C and vortexed for about 2hours. The mixture was dried and the remaining solid was analyzed by XPRD (see Figure 4B of WO2013010916, Form B).

Example 4.3: Preparation of phosphate salt of (R)-3-(6-(4-methylphenyl)-pyridin-3-yloxy)-1- aza-bicyclo[2.2.21octane in crystalline form (Form C) [reference]: 200mg of (R)-3-(6-(4-methylphenyl)-pyridtn-3-yloxy)-1-aza-bicyclo[2.2.23octane in free base form was suspended in 2ml ethanol. One-third of the stochiometric amount of phosphoric acid was added. The slurry obtained was stirred at ambient temperature and held for 14 hours. Solids were separated by filtration and analyzed by XPRD (see Figure 4C of WO2013010916, Form C).

Example 5.1: Preparation of mono-succinate salt of (R)-3-(6-(4-methylphenyl[3/4]-pyridin-3- yloxy)-1-aza-bicvclo-[2.2.2]octane in crystalline form (Form A) [reference]: 500 mg of (R)-3-(6-(4-methylphenyl)-pyridin-3-yloxy)-1-aza-bicyclo-[2.2.2]octane in free base form were suspended in 5 ml ethanol. A stochiometric amount of of succinic acid was added. The resulting solution was stirred at ambient tempertature for 14 hours. The precipitate was collected by filtration and analyzed by proton-NMR and XRPD (see Figure 5 of WO2013010916, Form A). Yield was 64%. Analysis of proton-NMR confirmed salt formation, a base/acid ratio of about 1: 1 and the fact that the salt was not a solvate.

Example 5.2: Preparation of anhydrous mono-succinate salt of (R)-3-(6-(4-methylphenyl[3/4]- pyridin-3-yloxy)-1 -aza-bicycio[2.2.21octane in crystalline form [reference]: When the Form A of the mono-succinate salt (see Example 5, 1) was subjected to heat (under nitrogen) from 25°C to 1 15°C, an anhydrous form of mono-succinate salt was observed and analyzed by XRPD (see Figure 5 of WO2013010916, Form B).

Example 6: Preparation of mono-malonate salt of (R)-3-(6-(4-methylphenyl)-pyridin-3-yloxy[3/4]- 1 -aza-bicvclo-[2.2.2]octane in crystalline form [reference]: 500 mg of (R)-3-(6-(4-methylphenyl)-pyridin-3-yloxy)-1-aza-bicycloE2.2.2]octane were suspended in 5 ml acetonitrile. A stochiometric amount of of malonic acid was added The resulting solution was stirred at ambient tempertature for 14 hours. The precipitate was collected by filtration and analyzed by proton-NMR and XRPD (see Figure 6 of WO2013010916). Yield was 77%. Analysis of proton-NMR confirmed salt formation, a base/acid ratio of about 1:1 and the fact that the salt was not a solvate.

### Example 7: Comparison of physico-chemical parameters of salt forms

| | Aqueous solubility [mg/ml] | Initial water content | Hygroscopy @ 85%RH | m.p. | Decomposition temperature | Known Multiple forms |
|---|---|---|---|---|---|---|
| Free Base | 0.05 | 0.1 | ~2 | 106 | 224 | Yes |
| Fumarate | >30 | <0.5 | 0.5 | 164 | 207 | No |
| Maleate | >30 | <0.1 | 0.3 | 154 | 207 | No |
| Hydrochloride, Form A | >30 | ~5 | ~5 | 240 | 262 | Yes |
| Phosphate, Form A | >30 | ~0.5 | 0.2 | 222 | 222 | Yes |
| Succinate | 2-15 | ~4.5 | 0.3 | 113 | 222 | Yes |
| Malonate | >30 | ~0 | 1.3 | 140 | 148 | No |

### Example 8: Hard Capsules

Hard gelatin capsules, each comprising as active ingredient 0.5, 5 or 25 mg of the mono- fumarate of (R)-3-(6-(4-methylphenyl)-pyridin-3-yloxy)-1-aza-bicyclo[2.2.2]octane can be prepared as follows:

| Ingredient for capsule fill | % (w/w) for 0.5 mg capsules | % (w/w) for 5 mg capsules | % (w/w) for 25 mg capsules |
|---|---|---|---|
| Mono-fumarate of (R)-3-(6-(4-methylphenyl)-pyridin-3-yloxy)-1-aza-bicyclo[2.2.2]octane | 0.46 | 4.65 | 23.23 |
| Lactose monohydrate | 65.24 | 61.05 | 42.47 |
| Microcrystalline cellulose | 25.00 | 25.00 | 25.00 |
| Hypromellose | 2.50 | 2.50 | 2.50 |
| Sodium croscarmellose | 6.00 | 6.00 | 6.00 |
| Colloidal silicon dioxide | 0.30 | 0.30 | 0.30 |
| Magnesium stearate | 0.50 | 0.50 | 0.50 |
| Purified water* | q.s. | q.s. | q.s. |

| | | | |
|---|---|---|---|
| * removed during processing | | | |

Preparation process: Mono-fumarate of (R)-3-(6-(4-methylphenyl)-pyridin-3-yloxy)-1-aza-bicyclo[2.2.2]octane, lactose monohydrate, microcrystalline cellulose, a portion of sodium croscarmellose and hypromellose are dry mixed in a high shear mixer bowl, and granulating fluid (purified water) added. Once the granulation is complete, the wet granules are dried in a fluid bed drier and the dry granules are milled. The remaining sodium croscarmellose and colloidal silicon dioxide are passed through a suitable sieve and added to the dried granular material and blended in a suitable blending shell. This is achieved by co-sieving the sodium croscarmellose and the colloidal silicon dioxide with a portion of the milled granules through a suitable sieve into the blending shell. Similarly, the required amount of sieved magnesium stearate is added to the bulk granule and then mixed in the same blending shell. This final blend is encapsulated into capsules using automated equipment. Weigth ratio of capsule fill to empty capsule shells is 2: 1.

### Example 9: Tablets

Example 9.1: Film-coated tablet: Film-coated tablets containing e.g. 0.5 mg of the mono-fumarate of (R)-3-(6-(4- methylphenyl)-pyridin-3-yloxy)-1-aza-bicyclo[2.2.2]octane may be prepared as follows: Preparation of pre-mix: Weigh-in mono-fumarate of (R)-3-(6-(4-methylphenyl)-pyridin-3-yloxy)-1-aza-bicyclo[2.2.2]octane (e.g. approx. 0.7%) and maize starch (e.g. approx. 13%), mix in a tumble blender (approx 100-300 rotations), pass through a sieve of approx. 0.25-1.0 mm mesh-size. Mix in a tumble blender (approx. 100-300 rotations). Preparation of final blend: To above pre-mix add microcrystalline cellulose (e.g. approx. 25%), sprayed lactose (e.g. approx. 68%), sodium-carboxymethylcellulose XL (e.g. approx. 2%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations). Add the sodium-stearyl-fumarate (e.g. approx. 1.5%) through a handsieve at approx. 0.5-1.0 mm mesh-size and mix in a tumble blender (approx. 30-150 rotations). Compression: On a rotary press compress the above final blend to cores of approx. 100mg, using the dosage specific tooling (e.g. approx. 6mm, round, curved). Coating: Prepare a suspension in water with basic coating premtxes black, red, yellow and/or white. Coat the above obtained cores in a perforated coating pan, and dry.

Example 9.2: Bilaver film-coated tablet: Bilayer film-coated tablets containing e.g. 2.5 mg of the mono-fumarate of (R)-3-(6-(4-methylphenyl)-pyridin-3-yloxy)-1-aza-bicyclo[2.2.2]octane may be prepared as follows: Final active blend: Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations). Add the Na-stearyl-fumarate (e.g. approx. 3%) through a handsieve at approx. 0.5-10mm and mix in a tumble blender (approx 30-150 rotations). Final placebo blend: Weigh-in microcrystalline cellulose (e.g. approx. 26%), sprayed lactose (e.g. approx. 69%), sodium-carboxymethylcellulose XL (e.g. approx. 1.9%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations). Add the sodium-stearyl-fumarate (e.g. approx. 3%) through a handsieve at approx. 0.5-1.0 mm and mix in a tumble blender (approx 30-150 rotations). Compression: On a rotary press compress the above final blends to a btlayer tablet-core of approx. 100mg with one placebo layer (approx. 77.5mg) and one active layer (approx. 22.5mg), using the dosage specific tooling (e.g. approx. 6mm, round, curved). Coating: Prepare a suspension in water with basic coating premixes black, red, yellow and/or white. Coat the above obtained cores in a perforated coating pan, and dry.

Example 9.3: Film-coated tablet: Film-coated tablets containing e.g. 50 mg of the mono-fumarate of (R)-3-(6-(4- methylphenyl)-pyridin-3-yloxy)-1-aza-bicyclo[2.2.2]octane may be prepared as follows: Final blend: Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations). Add the sodium-stearyl-fumarate (e.g. approx. 3%) through a handsieve at approx. 0.5-10mm and mix in a tumble blender (approx. 30-150 rotations). Compression: Compress the above final blend on a rotary press to cores, using the dosage specific tooling (e.g. approx. 15*5.9mm, round, curved). Coating: Prepare a suspension in water with basic coating premixes black, red, yellow and/or white. Coat the above obtained cores in a perforated coating pan, and dry.

### Example 10: Biological Data

The usefulness of (R)-3-(6-(4-methylphenyl)-pyridin-3-yloxy)-1-aza-bicyclo[2.2.2]octane in its various forms, e.g. in free base form (Compound A) or in mono-fumarate salt form (Compound B) in the treatment of the above-mentioned disorders can be confirmed in a range of standard tests including those indicated below.

10.1. In-vitro Tests: Selectivity of (R)-3-(6-(4-methylphenyl)-pyridin-3-yloxy[3/4]-1-aza- bicyclof2.2.21octane against a4B2-nAChR: Based on the activity/selectivity data shown below it is concluded that said compound is a selective agonist at the a7-nAChR.

| Compound | α7-nAChR activity | | α4β2-nAChR activity | | |
|---|---|---|---|---|---|
| | Potency EC₅₀ (nM) | Efficacy compared to epibatidine (100%) | IC₅₀ (nM) | EC₅₀ (nM) | fold selectivity |
| A | 35 | 75 | 5598 | >100'000 | 164 |

Assay: To assess a7-nAChR activity, a functional assay was employed using GH3 cells that recombinantly expressed human a7-nAChR. 40000 cells per well were seeded 48 h prior to the experiment on black 96-well plates (Costar) and incubated at 37°C in a humidified atmosphere (5 % C02/95 % air). On the day of the experiment, medium was removed by flicking the plates and replaced with 0.1 ml growth medium containing 0.002 mM Fluo-4, (Molecular Probes) in the presence of 2.5 mM probenecid (Sigma). The cells were incubated at 37°C in a humidified atmosphere (5 % C02/95 % air) for 1 h. Plates were flicked to remove excess of Fluo-4, washed twice with Hepes-buffered salt solution (HBSS, in mM: NaCI 130, KCI 5.4, CaCI2 2, MgS04 0.8, NaH2P04 0.9, glucose 25, Hepes 20, pH 7.4; HBS) and refilled with 0.1 ml of HBS containing antagonist when appropriate. The incubation in the presence of the antagonist lasted 3-5 minutes. Plates were placed in the cell plate stage of a FLIPR device (fluorimetric imaging plate reader, Molecular Devices, Sunnyvale, CA, USA). After recording of the baseline (laser: excitation 488 nm at 1 W, CCD camera opening of 0.4 seconds) the agonists (0.05 ml) were added to the cell plate using the FLIPR 96-tip pipettor while simultaneously recording the fluorescence. Calcium kinetic data were normalized to the maximal fitted response induced by epibatidine, which is a full agonist at a7-nAChR. Four parameter Hill equations were fitted to the concentration-response. Values of Emax (maximal effect in % compared to the epibatidine response) and EC50 (concentration producing half the maximal effect in [mu][Mu]) were derived from this fit. Assay described in: D Feuerbach et al, Neuropharmacology (2005), 48, 215-227. To assess the activity of the compound of the invention on the human neuronal nAChR [alpha]4[beta]2, a similar functional assay is carried out using a human epithelial cell line stably expressing the human [alpha]4[beta]2 subtype (Michelmore et at., Naunyn-Schmiedeberg's Arch. Pharmacol. (2002) 366, 235). 0.2. In-vivo Preclinical Tests

10.2.1. Oral bioavailability and brain penetration of (R[3/4]-3-(6-(4-methylphenyl[3/4]-pyridin-3- yloxy)-1-aza-bicyclo[2.2.21 octane in mice: Based on the pharmacokinetic data shown below it is concluded that the brain concentration of said compound in mice is beyond (or at least equal) to the compound's EC50 at the a7- nAChR for at least 4 hours following an acute oral dose of 30 pmol/kg of the compound in free base form.

**Compound A:**

| Administration | Time (hour) | Plasma (pmoles/ml±SD) | Brain (pmoles/g±SD) | Ratio Brain/plasma |
|---|---|---|---|---|
| 30 µmol/kg p.o. | 0.25 | 573 ± 234 | 4631 ± 1717 | 8 |
| 30 µmol/kg p.o. | 0.5 | 559 ± 143 | 11430 ± 3441 | 20 |
| 30 µmol/kg p.o. | 1 | 322 ± 135 | 14948 ± 4716 | 46 |
| 30 µmol/kg p.o. | 4 | 20 ± 16 | 1272 ± 715 | 62 |
| 30 µmol/kg p.o. | 8 | 3.4 ± 0.8 | 58 ± 27 | 17 |
| 30 µmol/kg p.o. | 24 | - | - | - |

Assay: Compounds were orally (30 pmol/kg) administered. Male mice (30-35g, OF1/ICstrain) were sacrificed at indicated time points after oral administration. Trunk- blood was collected in EDTA-containing tubes and the brain was removed and immediately frozen on dry ice. To 100 [mu][Iota] plasma 10 [mu][Iota] internal standard (1.0 pmol of a compound with solubility and ionization properties similar to test compounds) was added and extracted three times with 500 [mu][Iota] dichloromethane. The combined extracts were then dried under a stream of nitrogen and re-dissolved in 100 [mu][Iota] acetonitrile/water (70% acetonitrile). Brains were weighed and homogenized in water (1:5 w/v). Two 100 [mu][Iota] aliquots of each homogenate + 10 [mu][Iota] of internal standard (same standard as used for the plasma samples) were extracted three times with 500 [mu][Iota] dichloromethane and further processed as the plasma samples. Samples were separated on Beckmann high-performance liquid chromatography equipment system with an autosampler (Gilson 233XL). A 10 min linear gadient (10 - 70%) of acetonitrile containing 0.5 % (v/v) formic acid was used to elute the compounds from Nucleosil CC-125/2 C18 reversed phase (Machery&Nagel) column. The limit of detection (LOD), defined as the lowest concentration of the extracted standard sample with a signal to noise ratio of ~3.

10.2.2. Functional read-out of (R)-3-(6-f4-methylphenyl)-pyridin-3-yloxy)-1-aza-bicyclo[2.2.2]octane in Mice (Social Recognition Test): Based on the functional in-vivo data shown below it is concluded that oral dosing of said compound at relevant concentrations leads to a specific effect associated with a7-nAChR (i.e. cognition enhancement in the Social Recognition Test in mouse).

| Compound | Reduction in time scrutinizing in % ± SEM at 24 h | Dose In mg/kg |
|---|---|---|
| A | 36 ± 6 | 0.3 |

Assay: Social interactions between two experimental animals are influenced by their degree of familiarity: the better they know each other, the less time they spend on mutual scrutiny at each meeting. In agreement with published data in rats (Mondadoriet al., 1993) we have observed (i) that an adult mouse shows a shortened scrutiny of a young conspecific if the two mice are brought together again within a short time interval (e.g. 1 hour), (ii) that this curtailment is attributable to memory processes: it does not occur if the familiar young partner is replaced by a strange (unfamiliar) young mouse on the second occasion and (iii) that the adult mouse's recollection of the previously scrutinized juvenile partner fades with the elapsed time, i.e., after 24 h, scrutiny takes just about as long as at the first encounter. Memory enhancing agents (i.e. oxiracetam) facilitate learning to the extent that the previously met (familiar) partner is still remembered after 24 h, whereas in vehicle treated control animals the memory usualy fades after less than 1 hour (Thor and Holloway, 1982) or after 2-3 hours. Baseline-test: Pairs consisting of one adult and one young mouse were assigned at random to the experimental and control groups. In each pair only the adult mouse was orally treated 1 hour before the trial with either vehicle or the test compound. The duration of active contacts of the adult mouse with the young mouse was manually recorded over a period of 3 min, including the following behavioural, approach-related items: sniffing, nosing, grooming, licking, pawing and playing, anogenital exploration and orientation toward the young mouse; orientation, thereby, was defined as tip of nose of the adult mouse less than approximately 1 cm distant from the young mousds body. Re-test: Twenty-four hours after the baseline-test, the adults in each treatment group were confronted again with the previously encountered (familiar) partner, whereas the half of the adult animals were put together with the previously encountered (familiar) partner and the other half with another (unfamiliar) young mouse. Again the duration of active approach-behaviours was recorded during a 3-min period. Prior to re-test no oral injection was given. In the table the reduction in time scrutinizing the familiar partner at time 24 compared with the familiar partner at time 0 minutes is given (value of zero would signify no reduction).

10.2.3. Oral bioavailability of (R[3/4]-3-f6-(4-methylphenyl)-pyridin-3-yloxy)-1-aza- bicvcloi2.2.21octane in Dogs: Based on the pharmacokinetic data shown below it is concluded that the compound reaches significant blood levels in dogs following an acute oral dose of 1.4 pmol/kg of the compound in fumarate salt form.

**Compound B:**

| Administration | Time (hour) | Plasma (pmoles/ml±SD) |
|---|---|---|
| 1.4 µmol/kg p.o. | 0.25 | 14.7 ± 1.1 |
| 1.4 µmol/kg p.o. | 0.5 | 49.4 ± 21.9 |
| 1.4 µmol/kg p.o. | 1 | 67.6 ± 22.6 |
| 1.4 µmol/kg p.o. | 2 | 75.2 ± 36.8 |
| 1.4 µmol/kg p.o. | 4 | 27.5 ± 13.3 |
| 1.4 µmol/kg p.o. | 8 | 8.1 ± 3.0 |
| 1.4 µmol/kg p.o. | 24 | 0.8 ± 0.7 |

Assay: The compound was given to N=3 male beagle dogs in tritiated form: The concentration of the compound in blood was determined by LC-RID. The procedure involved the addition of 5 pg of the compound as internal standard (200 [mu][Iota]_ of solution containing 25 g/mL of the compound) to 1 mL of blood. After further addition of 1 mL of water, 0.1 mL of buffer pH 9 and 4 mL of tert-butylmethylether, the samples were shaken for 30 min and centrifuged (4000 g for 10 min at 22°C). The organic phase was transferred into a tube and evaporated in a Speedvac. The residue was reconstituted in 250 pL of mobile phase- water (80:20 v/v) followed by 75 [mu][iota] ofn-hexane and transferred into an autosampler vial. After centrifugation (13,000 g for 2 min at 22°C), the hexane layer was pipetted off and discarded, 200 [mu][iota] of the remainder was injected onto an RP18 column (Waters XTerra, 5 [mu][iota][eta], 3.9 x 150 mm at 40°C) to separate the compound from potential metabolites and endogenous compounds. The mobile phase of ammonium acetate (10 mM: 0.1 % v/v TFA- acetonitrile, 58:42 v/v) was used at a flow rate of 1.0 mL/min. The effluent was monitored by a UV-detector set at 261 nm. The peak corresponding to the unchanged compound was collected in a polyethylene vial by a fraction collector (SuperFrac, Pharmacia L B) and analyzed for radioactivity. The concentration of the compound in each sample was calculated from the ratio of the amount of radioactivity in the eiuate fraction to the area of the ultraviolet absorbance of the non-radiolabeled the compound, that was used as the internal standard.

10.2.4: Pharmacokinetics of (RV3-(6-(4-methylphenvn-pyridin-3-yloxy)-1-aza-bicyclo[2.2.21octane in rats: Dosing information in this Example 10.2.4 is given relative to the free form, (R)-3-{6-(4-methylphenyl)-pyridin-3-yloxy)-1-aza-bicyclo[2.2.2]octane, and is independent from the salt forming agent. If a salt forming agent is used (e.g. fumarate), a corresponding higher amount of the salt will be used to achieve the intended dosing. Based on the pharmacokinetic data shown below: After acute oral dosing of 10 mg/kg (R)-3-(6-(4-methylphenyl)-pyridin-3-yloxy)-1-aza-bicyclo[2.2.2]octane in mono-fumarate salt form, a Cmax of the free form in plasma of 161+-53 ng/ml was reached at 0.25-0.5h. The AUCIast amounted to 249+-42 h ng/ml. After acute oral dosing of 10 mg/kg (R)-3-(6-(4-methylphenyl)-pyridin-3-yloxy)-1-aza-bicyclo[2.2.2]octane in free form, a Cmax of the free form in plasma of 1 12+-40 ng/ml was reached at 0.25-0.5h. The AUCIast amounted to 200+-62 h ng/ml. Pharmacokinetics of (R)-3-(6-(4-methylphenyl)-pyridin-3-yloxy)-1-aza-bicyclo[2,2,2]octane in Wistar rats after oral administration of 10 mg/kg of the compound in mono-fumarate salt form (Compound B) and in free base form (Compound A) were assessed. Both compounds were dissolved in Klucel(TM) (0.5% in water) and were orally administered by gavage (5 ml/kg) to conscious rats (n=8, crossover design). K3-EDTA blood (-0.2 ml) was collected by puncture of a sublingual vein under light isoflurane anesthesia at the following time points: 0.25, 0.5, 1, 2, 3, 4, 6, 8, 24 h post dose. Immediately after sampling, blood samples were put on ice and were processed to plasma by centrifugation at 4<D>C within 15 min after sampling (1000g, 10 min, 4°C) to obtain -100 [mu][Iota] plasma/sample. All plasma samples were stored at -80°C until analysis. Since the PK study was performed in a crossover design, a recovery period of approximately 2 weeks took place before the next treatment/blood sampling was performed. The determination of compounds in plasma was done by LC-MS/MS using electrospray ionization.

**a. Concentrations (ng/ml) and derived pharmacokinetic parameters of (R)-3-(6-(4-methylphenyl)-pyridin-3-yloxy)-1-aza-bicyclo[2.2.2]octane in plasma of male Wistar rats following oral administration of 10 mg/kg free base**

| Phase | 1 | 1 | 1 | 1 | 2 | 2 | 2 | 2 | | | |
|---|---|---|---|---|---|---|---|---|---|---|---|
| Time (h) | Rat 01 | Rat 02 | Rat 03 | Rat 04 | Rat 05 | Rat 06 | Rat 07 | Rat 08 | Mean All | SD All | CV (%) |
| 0.25 | 58.8 | 89.6 | 83.0 | 79.4 | 105 | 188 | 49.0 | 145 | 100 | 46.2 | 46.3 |
| 0.5 | 34.8 | 51.2 | 64.5 | 94.8 | 119 | 122 | 117 | 65.0 | 83.5 | 34.0 | 40.7 |
| 1 | 20.1 | 31.4 | 63.9 | 36.0 | 65.8 | 83.6 | 87.0 | 33.8 | 52.7 | 25.6 | 48.5 |
| 2 | 6.79 | 12.6 | 51.7 | 16.2 | 43.2 | 46.9 | 42.2 | 29.9 | 31.2 | 17.3 | 55.5 |
| 3 | 5.31 | 8.38 | 26.3 | 7.84 | 23.0 | 34.4 | 18.4 | 6.20 | 16.2 | 10.9 | 67.3 |
| 4 | 3.94 | 3.99 | 18.2 | 4.54 | 8.63 | 21.1 | 13.8 | 8.55 | 10.3 | 6.66 | 64.4 |
| 6 | 4.87 | 1.81 | 6.62 | 1.27 | 5.32 | 4.38 | 4.59 | 6.06 | 4.37 | 1.90 | 43.5 |
| 8 | 6.62 | 0.812 | 2.46 | 1.32 | 3.67 | 1.54 | 2.25 | 9.67 | 3.54 | 3.08 | 86.9 |
| 24 | 1.40 | 2.44 | 0.547 | 1.02 | 0* | 0.632 | 0.248 | 0.287 | 0.822 | 0.792 | 96.4 |
| Body weight (kg)^{b} | 0.297 | 0.277 | 0.288 | 0.271 | 0.309 | 0.318 | 0.315 | 0.350 | --- | --- | --- |
| Dose (mg/kg) | 10.1 | 10.2 | 9.76 | 10.1 | 9.88 | 9.96 | 10.0 | 9.65 | 10.0 | 0.184 | 1.85 |
| Tmax (h) | 0.25 | 0.25 | 0.25 | 0.50 | 0.50 | 0.25 | 0.50 | 0.25 | 0.25 | [0.25-0.50]^{a} | |
| Tlast (h) | 24.0 | 24.0 | 24.0 | 24.0 | 8.0 | 24.0 | 24.0 | 24.0 | 24.0 | [8.0-24.0]^{a} | |
| Cmax (ng/mL) | 58.8 | 89.6 | 83.0 | 94.8 | 119 | 188 | 117 | 145 | 112 | 40.4 | 36.1 |
| Cmax/Dose (ng/mL)/(mg/kg) | 5.81 | 8.79 | 8.50 | 9.40 | 12.0 | 18.9 | 11.7 | 15.0 | 11.3 | 4.14 | 36.7 |
| AUClast (h·ng/mL) | 141 | 123 | 238 | 136 | 214 | 296 | 234 | 236 | 200 | 61.9 | 30.6 |
| AUClast/Dose (h·ng/mL)/(mg/kg) | 14.0 | 12.0 | 24.4 | 13.5 | 21.6 | 29.7 | 23.4 | 24.5 | 20.4 | 6.43 | 31.5 |
| T1/2 (h) | 8.67 | nd | 5.71 | nd | 3.24 | nd | 4.54 | 3.67 | 5.17 | 2.17 | 42.1 |
| T1/2 range (h) | [6-24] | nd | [6-24] | nd | [4-8] | nd | [6-24] | [6-24] | | | |

| | | | | | | | | | | | |
|---|---|---|---|---|---|---|---|---|---|---|---|
| ^{a}: Median [range] ^{b}: At time of treatment nd: Not determined due to r² <0.75 or AUC%extrapolated>30%. * Values BLOQ (0.150 ng/mL) were set to 0 for Phoenix PK calculations. | | | | | | | | | | | |

**b. Concentrations (ng/ml) and derived pharmacokinetic parameters of (R)-3-(6-(4-methylphenyl)-pyridin-3-yloxy)-1-aza-bicyclo[2.2.2]octane in plasma of male Wistar rats following oral administration of 10 mg/kg mono-fumarafe.**

| Phase | 2 | 2 | 2 | 2 | 1 | 1 | 1 | 1 | | | |
|---|---|---|---|---|---|---|---|---|---|---|---|
| Time (h) | Rat 01 | Rat 02 | Rat 03 | Rat 04 | Rat 05 | Rat 06 | Rat 07 | Rat 08 | Mean All | SD All | CV (%) |
| 0.25 | 167 | 162 | 77.8 | 89.0 | 242 | 204 | 93.3 | 153 | 149 | 58.5 | 39.4 |
| 0.5 | 104 | 130 | 78.4 | 99.7 | 143 | 179 | 184 | 138 | 132 | 37.4 | 28.3 |
| 1 | 67.1 | 88.8 | 41.3 | 62.8 | 83.2 | 80.8 | 101 | 94.2 | 77.4 | 19.4 | 25.1 |
| 2 | 27.4 | 39.2 | 37.8 | 24.6 | 47.1 | 37.6 | 54.8 | 42.6 | 38.9 | 9.81 | 25.2 |
| 3 | 12.4 | 17.7 | 25.3 | 11.7 | 19.6 | 20.8 | 25.1 | 23.2 | 19.5 | 5.27 | 27.1 |
| 4 | 9.06 | 10.6 | 13.3 | 5.90 | 9.71 | 11.8 | 13.0 | 11.1 | 10.6 | 2.39 | 22.6 |
| 6 | 7.49 | 3.47 | 763 | 5.79 | 4.29 | 4.24 | 4.05 | 3.87 | 5.10 | 1.66 | 32.5 |
| 8 | 2.91 | 2.35 | 3.62 | 2.62 | 2.46 | 1.94 | 2.38 | 2.07 | 2.54 | 0.529 | 20.8 |
| 24 | 0* | 0.276 | 0.210 | 0.169 | 0* | 1.40 | 0.162 | 0.344 | 0.320 | 0.453 | 141 |
| Body weight (kg)^{b} | 0.341 | 0.315 | 0.330 | 0.300 | 0.268 | 0.266 | 0.276 | 0.290 | --- | --- | --- |
| Dose (mg/kg) | 9.73 | 9.86 | 9.95 | 9.82 | 9.61 | 9.69 | 10.1 | 9.77 | 9.81 | 0.146 | 1.49 |
| Tmax (h) | 0.25 | 0.25 | 0.50 | 0.50 | 0.25 | 0.25 | 0.50 | 0.25 | 0.25 [0.25-0.50]^{a} | | |
| Tlast (h) | 8.0 | 24.0 | 24.0 | 24.0 | 8.0 | 24.0 | 24.0 | 24.0 | 24.0 [8.0-24.0]^{a} | | |
| Cmax (ng/mL) | 167 | 162 | 78.4 | 99.7 | 242 | 204 | 184 | 153 | 161 | 52.9 | 32.8 |
| Cmax/D (ng/mL)/(mg/kg) | 17.2 | 16.4 | 7.88 | 10.2 | 25.2 | 21.1 | 18.3 | 15.7 | 16.5 | 5.54 | 33.6 |
| AUClast (h·ng/mL) | 202 | 259 | 212 | 188 | 269 | 292 | 298 | 272 | 249 | 42.2 | 16.9 |
| AUClast/D (h·ng/mL)/(mg/kg) | 20.8 | 26.3 | 21.4 | 19.2 | 28.0 | 30.1 | 29.6 | 27.9 | 25.4 | 4.30 | 16.9 |
| T1/2 (h) | 2.44 | 5.02 | 3.62 | 3.71 | 2.02 | nd | 3.97 | 5.51 | 3.76 | 1.26 | 40.3 |
| T1/2 range (h) | [4-8] | [6-24] | [6-24] | [6-24] | [4-8] | nd | [6-24] | [6-24] | | | |

| | | | | | | | | | | | |
|---|---|---|---|---|---|---|---|---|---|---|---|
| ^{a}: Median [range] ^{b}: At time of treatment nd: Not determined due to r² <0.75. * Values BLOQ (0.150 ng/mL) were set to 0 for Phoenix PK calculations. | | | | | | | | | | | |

Example 11: Preparation of 5-chloro-2-(4-methylphenyl)pyridine (Process according to Section A): Under nitrogen 2,5-dichloro-pyridine (40g, 270 mmol), 4-methylphenylboronic acid (39 g, 289 mmol) and bistriphenylphosphin-palladium(II) dichloride (1.14g; 1.6mmol) were suspended in water (258g) / THF (117g) for approx. 30 min at 35-55°C. A solution of tripotassium phosphate (143.4g, 676 mmol) in water (143g) was added at 35-55X during approx. 60- 120 min and 55°C was maintained for another approx. 30- 45 min. More tripotassium phosphate (22.9g, 108mmol) in water (22.9g) was added over a period of approx. 30 min and the temperature was raised to 55-60°C to complete the reaction within another approx. 2h. For extractive palladium removal a solution of cysteine (ca.16g) in water (115 g) was added to the reaction mixture at 60-55°C. After approx. 1 h at 55°C the biphasic reaction mixture was clarified by filtration over a pad of cellflock filter aid (2-5g) and a THF/water mixture (110 g/75g) was used for rinsing. The layers of the combined filtrates were separated at 25°C and the salt containing water layer was extracted with THF (1x57g). The combined THF layers were diluted with ethanol 94% (195g) and concentrated by distillation under reduced pressure (300-200mbar) at a jacket temperature of 45°C in order to remove the bulk of THF (175-250g). To the remaining product solution further ethanol (97g) was added and at 45- 55°C water (565g) was gradually added over a period of approx. 60min to induce and maintain crystallization. After 30 min the temperature was lowered to approx. 20°C in approx. 90 - 120 min and after another hour at that temperature the solids were collected by filtration, washed with ethanol/water 1:2 and dried under reduced pressure to yield 5-chloro-2-(4- methylphenyl)pyridine (52.5g; 95% of theory; purity>95%; Pd <25ppm).

### Example 12: Preparation of (R)-3-(6-(4-methylphenyl)-pyridin-3-yloxy[3/4]-1-aza- bicvclo[2.2.21octane in free form and fumarate salt form (Process according to Section B)

Example 12.1: Formation of free form: Under nitrogen, to 3R-quinuclidinol (43.8g, 0.34mol) in DMSO (792g) an approx. 20% THF solution of potassium tert-butoxide (21 Og, 0.375mol) was added and at approx. 40-45°C under reduced pressure the THF solvent was distilled off. The temperature of the reaction mixture was raised to 90°C and the solid chloro-2-(4-methylphenyl)pyridine (61.2g, 0.30mol) was gradually added in at least 4 portions. The temperature was raised further to appux. 100-105°C and after at least another 3 hours at this temperature the reaction to (R)- 3-(6-(4-methylphenyl)-pyridin-3-yloxy)-1-aza-bicyclo[2.2.2]octane was complete. Water (150 g) was added to the reaction mixture at 60-25°C and the temperature was gradually lowered to approx. 20°C in approx. 60 min and additional water (21 Og) was added. After at least another 2 further hours at this temperature the fine solids were collected by filtration, washed successively with DMSO /water (approx. 322g; 2 1 mixture), water (500g) and water/ethanol (approx. 500g 9:1 mixture) and dried at 60°C under reduced pressure to yield (R)-3-(6-(4-methylphenyl)-pyridin-3-yloxy)-1-aza-bicyclo[2.2.2]octane (56.3 g, 63% of theory).

Example 12.2: Formation of fumarate salt form: To a clear solution of (R)-3-(6-(4-methylphenyl)-pyridin-3-yloxy)-1-aza-bicyclo[2.2.2]octane (39.6g; 0.135 mol) and fumaric acid (16.4g, 0.141 mol) in ethanol (330g) / water (21g) at 65°C tert. butylmethylether (142.5g) was addded and the reaction mixture was cooled to 23°C in approx. 60min. Further tert-butylmethylether (170.6g) was added. After at least another 2 hours the solids were collected by filtration, washed with ethanol/tert.butylmethylether (153 g; 1.1 mixture) and dried at 55-60°C under reduced pressure to yield (R)-3-(6-(4-methylphenyl)-pyridin-3-y!oxy)-1 -aza-bicyclo[2.2.2]octane hydrogenfumarate (43.8g, 79% of theory).

Example 13: Preparation of {R)-3-(6-(4-methylphenyl)-pyridin-3-yloxy)-1-aza-bicyclo[2.2.21octane in free form and fumarate salt form

Example 13.1: Formation of free form: Under nitrogen to 3R-quinuclidinol (41 4g, 0.325mol) in DMSO (320g) a solution of 5-chloro- 2-(4-methylphenyl)pyridine (51 g, 0.250mol) in toluene (201 g) was added. The temperature was raised gradually to approx. 100-105°C while residual water, if any, was removed by refluxing under reduced pressure at a water trap for ca. 45 min. Over a period of approx. 90 min an approx. 20% THF solution of potassium tert-butoxide (158.8g, 0.283mol) was continuously added while gradually the THF solvent distilled off. After another 2-5 hours at approx. 100-105°C the reaction to (R)-3-(6-(4-methylphenyl)-pyridin-3-yloxy)-1-aza-bicyclo[2.2.2]octane was complete. Water (293g) was added to the reaction mixture at 60-25°C. The layers were separated and the toluene layer was washed with water (2x42g). The toluene solution was dried at ca. 60°C by refluxing under reduced pressure at a water trap for ca. 45-60 min.

Example 13.2: Formation of fumarate salt form: To the toluene solution of Example 13.1 , at ca. 50-55°C, a slurry of fumaric acid (26.1g, 0.9eq) in EtOH 94% (22g) and toluene (97g) was gradually added. Further toluene (97g) was added for rinsing and after another ca, 30-60 min at 55°C the temperature was gradually lowered to approx. 20°C in approx. 120-180 min. After at least another 1 hour the solids were collected by filtration, washed with water saturated toluene (2x104g) and dried at 60°C under reduced pressure to yield (R)-3-(6-(4-methylphenyl)-pyridin-3-yloxy)-1-aza-bicyclo[2.2.2]octane hydrogenfumarate (84,8g; 82% of theory, based on amount of 5-chloro- 2-(4-methyiphenyl)pyridine used in Example 13.1).

Futility: The salts of the invention exhibit valuable pharmacological properties administered to animals/humans, and are therefore useful as pharmaceuticals. Salts of the invention are selective 7-nAChR partial agonists. Due to their pharmacological profiles, salts of the invention are anticipated to be useful for the treatment of diseases or conditions as diverse as CNS related diseases, PNS related diseases, diseases related to inflammation, pain and withdrawal symptoms caused by an abuse of chemical substances. Diseases or disorders related to the CNS include general anxiety disorders, cognitive disorders, learning and memory deficits and dysfunctions, Alzheimer's disease (AD), prodromal AD, mild cognitive impairment in the elderly (MCI), amnestic MCI, age associated memory impairment, attention deficit and hyperactivity disorder (ADHD), Parkinson's disease, L-dopa induced dyskinesias associated with Parkinson's disease, Huntington's disease, ALS, prionic neurodegenerative disorders such as Creutzf eld- Jacob disease and kuru disease, Gilles de la Tourette's syndrome, psychosis, depression and depressive disorders, mania, manic depression, schizophrenia, the cognitive deficits in schizophrenia, obsessive compulsive disorders, panic disorders, eating disorders, nociception, AIDS-dementia, senile dementia, mild cognitive dysfunctions related to age, autism, dyslexia, tardive dyskinesia, epilepsy, and convulsive disorders, post-traumatic stress disorders, transient anoxia, pseudodementia, premenstrual syndrome, late luteal phase syndrome and jet lag. Furthermore, salts of the invention maybe useful for the treatment of endocrine disorders, such as thyrotoxicosis, pheochromocytoma, hypertension and arrhythmias as well as angina pectoris, hyperkinesia, premature ejaculation and erectile difficulty. Still further, salts of the invention may be useful in the treatment of inflammatory disorders (Wang et al., Nature 2003, 421, 384; de Jonge et al., Nature Immunology 2005, 6, 844; Saeed et al., JEM 2005, 7, 11 13), disorders or conditions including inflammatory skin disorders, rheumatoid arthritis, post-operative ileus, Crohn's diesease, inflammatory bowel disease, ulcerative colitis, sepsis, fibromyalgia, pancreatitis and diarrhoea. Salts of the invention may further be useful for the treatment of withdrawal symptoms caused by termination of the use of addictive substances, like heroin, cocaine, tobacco, nicotine, opioids, benzodiazepines and alcohol. Furthermore, salts of the invention may be useful for the treatment of pain, e.g. caused by migraine, postoperative pain, phantom limb pain or pain associated with cancer. The pain may comprise inflammatory or neuropathic pain, central pain, chronic headache, pain related to diabetic neuropathy, to post therapeutic neuralgia or to peripheral nerve injury. Furthermore, degenerative ocular disorders which may be treated include ocular diseases which may directly or indirectly involve the degeneration of retinal cells, including ischemic retinopathies in general, anterior ischemic optic neuropathy, all forms of optic neuritis, age-related macular degeneration (AMD), in its dry forms (dry AMD) and wet forms (wet AMD), diabetic retinopathy, cystoid macular edema (CME), retinal detachment, retinitis pigmentosa, Stargardt's disease, Best's viteiiiform retinal degeneration, Leber's congenital amaurosis and other hereditary retinal degenerations, pathologic myopia, retinopathy of prematurity,and Leber's hereditary optic neuropathy. The salts of the invention can be combined with at least one compound selected from the group consisting of (a) conventional antipsychotics and (b) atypical antipsychotics, in which the antipsychotic is present in free form or in the form of a pharmaceutically acceptable salt; for simultaneous, separate or sequential use to treat psychiatric disorders. The term "psychiatric disorders" as used herein includes, but is not limited to schizophrenia, anxiety disorders, depression and bipolar disorders. Preferably, the psychiatric disorder is schizophrenia, more preferably schizophrenia which is refractory to monotherapy employing one of the combination partners alone. The term "conventional antipsychotics" as used herein includes, but is not limited to haloperidol, fluphenazine, thiotixene and flupentixol. The term "atypical antipsychotics" as used herein includes, but is not limited to clozaril, risperidone, olanzapine, quetiapine, ziprasidone and aripiprazol.

The salts of the invention are useful in the treatment of the above diseases/conditions.

Consequently, the invention also relates to a salt of the invention (e.g. the mono-fumarate salt of (R)-3-(6-(4-methylphenyl)-pyridin-3-yloxy)-1-aza-bicyclo[2.2.2]octane in crystalline form) for use as a medicament. In another embodiment, the invention also relates to a salt of the invention (e.g. the mono-fumarate salt of (R)-3-(6-(4-methylphenyl)-pyridin-3-yloxy)-1-aza-bicyclo[2.2.2]octane in crystalline form) for use in the prevention, treatment and / or delay of progression of a disease or condition, in which oc7-nAChR activation plays a role or is implicated. In another embodiment, the invention also relates to a salt of the invention (e.g. the mono-fumarate salt of (R)-3-(6-(4-methylphenyl)-pyridin-3-yloxy)-1-aza-bicyclo[2.2.2]octane in crystalline form) for use in the prevention, treatment and / or delay of progression of a psychiatric or neurodegenerative disorder. In another embodiment, the invention also relates to the use of a salt of the invention (e.g. the mono-fumarate salt of (R)-3-(6-(4-methylphenyl)-pyridin-3-yloxy)-1-aza-bicyclo[2.2.2]octane in crystalline form) for the manufacture of a medicament for the prevention, treatment and / or delay of progression of a disease or condition, in which ot7-nAChR activation plays a role or is implicated. In another embodiment, the invention also relates to the use of a salt of the invention (e.g. the mono-fumarate salt of (R)-3-(6-(4-methylphenyl)-pyridin-3-yloxy)-1-aza-bicyclo[2.2.2]octane in crystalline form) for the manufacture of a medicament for the prevention, treatment and / or delay of progression of a psychiatric or neurodegenerative disorder. In another embodiment, the invention also relates to a method for the prevention, treatment and / or delay of progression of a disease or condition, in which 7-nAChR activation plays a role or is implicated, in a subject in need of such treatment, which comprises administering to such subject a therapeutically effective amount of a salt of the invention (e.g. the mono-fumarate salt of (R)-3-(6-(4-methylphenyl)-pyridin-3-yloxy)-1-aza-bicyclo[2.2.2]octane in crystalline form). In another embodiment, the invention relates to a method for the prevention, treatment and / or delay of progression of a psychiatric or neurodegenerative disorder in a subject in need of such treatment, which comprises administering to such subject a therapeutically effective amount of a salt of the invention (e.g. the mono-fumarate salt of (R)-3-(6-(4-methylphenyl)-pyridin-3-yloxy)-1-aza-bicyclo[2.2.2]octane in crystalline form). In another embodiment, the invention relates to a method for the prevention, treatment and / or delay of progression of a disease or condition, in which a7-nAChR activation plays a role or is implicated, in a subject in need thereof, which comprises (i) diagnosing said disease or condition in said subject and (ii) administering to said subject a therapeutically effective amount of a salt of the invention (e.g. the mono-fumarate salt of (R)-3-(6-(4-methylphenyl)-pyridin-3-yloxy)-1-aza-bicyclo[2.2.2]octane in crystalline form).

In another embodiment, the invention relates to a method for the prevention, treatment and / or delay of progression of a psychiatric or neurodegenerative disorder, in a subject in need thereof, which comprises (i) diagnosing said disorder in said subject and (ii) administering to said subject a therapeutically effective amount of a salt of the invention (e.g. the mono-fumarate salt of (R)-3-(6-(4-methylphenyl)-pyridin-3-yloxy)-1-aza-bicyclo[2.2.2]octane in crystalline form). Treatment may comprise a reduction in the characteristics associated with the disease, condition or disorder, including, although not limited to, e.g. for schizophrenia: reduction in positive symptoms, negative symptoms, mood symptoms and/or cognitive symptoms and/or reduction in impulsive or violent behaviour. In the case of prophylactic treatment, the salt of the invention may be used to delay or prevent the onset of the Instant Movement Disorder. The term "subject" as used herein refers preferably to a human being, especially to a patient being diagnosed with the the disease, condition or disorder. The term "therapeutically effective amount" as used herein typically refers to a drug amount which, when administered to a subject, is sufficient to provide a therapeutic benefit, e.g. is sufficient for treating, preventing or delaying the progression of the disease, condition or disorder (e.g. the amount provides an amelioration of symptoms, e.g. it leads to a reduction of positive symptoms in schizophrenic patients). For the above-mentioned indications (the diseases, conditions and/or disorders) the appropriate dosage will vary depending upon, for example, the host, the mode of administration and the nature and severity of the condition being treated. However, in general, satisfactory results in animals are indicated to be obtained at a daily dosage of from about 0.01 to about 100 mg/kg body weight, preferably from about 0.1 to about 10 mg/kg body weight, e.g. 1 mg/kg. In larger mammals, for example humans, an indicated daily dosage is in the range from about 0.1 to about 1000 mg, preferably from about 1 to about 400 mg, most preferably from about 3 to about 100 mg of a salt of the invention conveniently administered, for example, in divided doses up to four times a day. Amorphous forms/crystalline forms of salts of the invention are useful as intermediates for preparing crystalline forms/other crystalline forms of salts of the invention that are useful in the treatment of the above diseases/conditions.

The invention relates to a blister packaging comprising an administration unit comprising fumarate salt of (R)-3-(6-(4-methylphenyl)-pyridin-3-yloxy)-1-aza-bicyclo[2.2.2]octane. The administration unit which is contained in the blister packaging according to the invention is useful for treating various diseases and disorders which include but are not limited to prevention, treatment of a disease, in which nAChR ct7 activation plays a role, revention, treatment of of psychiatric or neurodegenerative disorders, CNS. PNS diseases, and inflammation.

Preferably, the administration unit which is contained in the blister packaging according to the invention is solid, semisolid or liquid.

Preferably, the administration unit which is contained in the blister packaging according to the invention is for administration once daily, or twice daily, or thrice daily, or four times daily, or five times daily, or six times daily.

Preferably, the administration unit which is contained in the blister packaging according to the invention is monolithic or multiparticulate.

Preferably, the administration unit which is contained in the blister packaging according to the invention has a total weight of 10 mg to 3 g. Preferably, the administration unit which is contained in the blister packaging according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit which is contained in the blister packaging according to the invention is for systemic or topical administration.

In another preferred embodiment, the administration unit which is contained in the blister packaging according to the invention is for parenteral administration.

Preferably, the administration unit which is contained in the blister packaging according to the invention is for oral, buccal, opthalmic, nasal, rectal, transmucosal, or intestinal administration. Preferably, it is for oral administration.

Preferably, the administration unit which is contained in the blister packaging according to the invention is selected from the group consisting of tablets, capsules, effervescent tablets, orodispersible tablets, dragees, sachets, drops, suspensions, and powders. Preferably, it is a tablet.

Preferably, the administration unit which is contained in the blister packaging according to the invention is round or oblong; and/or is planar or biconvex.

In a preferred embodiment, the administration unit which is contained in the blister packaging according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit which is contained in the blister packaging according to the invention is oblong and has an aspect ratio of at least 1.1:1. Preferably, the aspect ratio is at least 4:3, or at least 21/2:1, or at least 3:2, or at least 16:10, or at least Psi:1, or at least 5:3, or at least 16:9.

In a preferred embodiment, the administration unit which is contained in the blister packaging according to the invention has been manufactured by direct compression.

In another preferred embodiment, the administration unit which is contained in the blister packaging according to the invention has been manufactured by granulation and subsequent compression of granules. Preferably, granulation is wet granulation or dry granulation.

Preferably, the administration unit which is contained in the blister packaging according to the invention has been compacted at a pressure of 10 MPa to 10,000 MPa. Preferably, the administration unit which is contained in the blister packaging according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

In a preferred embodiment, the administration unit which is contained in the blister packaging according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit which is contained in the blister packaging according to the invention, the film coating comprises a cellulose ether (e.g. hydroxypropylmethyl cellulose) or a synthetic polymer (e.g. polyvinyl alcohol).

In a preferred embodiment of the administration unit which is contained in the blister packaging according to the invention, the film coating has an average thickness of 0.01 µm to 0.03 µm, or 0.03 to 0.05 µm, or 0.05 to 0.1 µm, or 0.1 to 0.25 um, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 um, or 25 µm to 50 µm, or 50 µm to 100 urn, or 100 µm to 250 µm, or 250 µm to 500 µm, or 500 µm to 1000 µm.

In a preferred embodiment, the administration unit which is contained in the blister packaging according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59, 688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit which is contained in the blister packaging according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit which is contained in the blister packaging according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit which is contained in the blister packaging according to the invention has an average pore size of 0.001 µm to 1000 µm. Preferably, it has an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 µm, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

The pore size distribution of a tablet may be assessed by methods that are known to the skilled artisan and include but are not limited to gas adsorption (e.g. Stanley-Wood, N.G., Johansson, M.E., 1980. Variation of intra- and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra- and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 µm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit which is contained in the blister packaging according to the invention has a pore size distribution such that at least 10 % of the pores have a pore size of not more than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 0.1 µm.

In another preferred embodiment, the administration unit which is contained in the blister packaging according to the invention has a pore size distribution such that at least 10 % of the pores have a pore size of not less than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 µm.

In preferred embodiment, the administration unit which is contained in the blister packaging according to the invention has a pore size distribution such that at least 10 % of the pores have a pore size of not more than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 1 µm.

In another preferred embodiment, the administration unit which is contained in the blister packaging according to the invention has a pore size distribution such that at least 10 % of the pores have a pore size of not less than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 µm.

In a preferred embodiment, the administration unit which is contained in the blister packaging according to the invention has a pore size distribution such that at least 10 % of the pores have a pore size of not more than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 µm.

In another preferred embodiment, the administration unit which is contained in the blister packaging according to the invention has a pore size distribution such that at least 10 % of the pores have a pore size of not less than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 µm.

In a preferred embodiment, the administration unit which is contained in the blister packaging according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit which is contained in the blister packaging according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit which is contained in the blister packaging according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit which is contained in the blister packaging according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit which is contained in the blister packaging according to the invention is a capsule. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit which is contained in the blister packaging according to the invention, the capsule material comprises hard gelatine.

In a preferred embodiment, the administration unit which is contained in the blister packaging according to the invention passes the storage stability test according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit which is contained in the blister packaging according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit which is contained in the blister packaging according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit which is contained in the blister packaging according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit which is contained in the blister packaging according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit which is contained in the blister packaging according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit which is contained in the blister packaging according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit which is contained in the blister packaging according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit which is contained in the blister packaging according to the invention, the glidant is selected from the group consisting of fumed silica, cornstarch, talc, and magnesium carbonate.

In a preferred embodiment of the administration unit which is contained in the blister packaging according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit which is contained in the blister packaging according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit which is contained in the blister packaging according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit which is contained in the blister packaging according to the invention passes the test uniformity of content of single-dose preparations according to Ph. Eur.

In a preferred embodiment, the administration unit which is contained in the blister packaging according to the invention is for storage not above 50 °C. Preferably, it is for storage not above 25 °C.

The invention also relates to the administration unit which is contained in the blister packaging according to the invention for use in the treatment of a disorder or a disease in a mammal. Preferably, the mammal is a human. Preferably, the mammal is an adult. Preferably, the mammal does not suffer from liver damage and/or kidney damage.

Preferably, the mammal is not pregnant.

In a preferred embodiment, the administration unit which is contained in the blister packaging according to the invention is administered orally with a liquid. Preferably, the liquid is water, milk, juice or lemonade.

The invention relates to a blister packaging comprising one or more administration units according to the invention.

In a preferred embodiment, the blister packaging according to the invention comprises a material selected from the group consisting of paper, cardboard, paperboard, metal foil and plastic foil.

The packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

In a preferred embodiment, the blister packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the blister packaging according to the invention, the administration units do not substantially differ in at least one property selected from the group consisting of nature of excipients, content of excipients, water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

## Claims

1. A blister packaging comprising an administration unit, wherein the administration unit comprises a fumarate salt of (R)-3-(6-(4-methylphenyl)-pyridin-3-yloxy)-1-aza-bicyclo-[2.2.2]octane; and
wherein the administration unit is for use in the treatment of a disorder or a disease in a human, wherein the administration unit is administered once daily, or twice daily, or thrice daily.

2. The blister packaging according to claim 1, wherein the fumarate salt of (R)-3-(6-(4-methylphenyl)-pyridin-3-yloxy)-1-aza-bicyclo[2.2.2]octane is the mono-fumarate salt of (R)-3-(6-(4-methylphenyl)-pyridin-3-yloxy)-1-aza-bicyclo[2.2.2]octane.

3. The blister packaging according to claim 2, wherein the mono-fumarate salt of (R)-3-(6-(4-methylphenyl)-pyridin-3-yloxy)-1-aza-bicyclo[2.2.2]octane in crystalline form is **characterized by** an XRPD pattern with at least four of the following peaks at an angle of refraction 2 theta (20) of 3.8, 13.7, 15.2, 17.4, 19.8 and 20.1, ±0.2, respectively.

4. The blister packaging according to any of claims 1 to 3, wherein the administration unit is a tablet.

5. The blister packaging according to claim 4, wherein the tablet is round or oblong; and/or wherein the tablet is planar or biconvex.

## Patentansprüche

1. Eine Blisterverpackung umfassend eine Verabreichungseinheit, wobei die Verabreichungseinheit ein Fumarat-Salz von (R)-3-(6-(4-Methylphenyl)-pyridin-3-yloxy)-1-aza-bicyclo[2.2.2]octan umfasst; und
wobei die Verabreichungseinheit zur Anwendung bei der Behandlung einer Störung oder Erkrankung eines Menschen ist, bei der die Verabreichungseinheit einmal täglich, oder zweimal täglich, oder dreimal täglich verabreicht wird.

2. Die Blisterverpackung nach Anspruch 1, wobei das Fumarat-Salz von (R)-3-(6-(4-Methylphenyl)-pyridin-3-yloxy)-1-aza-bicyclo[2.2.2]octan das Mono-Fumarat-Salz von (R)-3-(6-(4-Methylphenyl)-pyridin-3-yloxy)-1-aza-bicyclo[2.2.2]octan ist.

3. Die Blisterverpackung nach Anspruch 2, wobei das Mono-Fumarat-Salz von (R)-3-(6-(4-Methylphenyl)-pyridin-3-yloxy)-1-aza-bicyclo[2.2.2]octan in kristalliner Form durch ein XRPD Muster gekennzeichnet ist mit mindestens vier der folgenden Peaks bei einem Refraktionswinkel 2 Theta (20) von 3,8, 13,7, 15,2, 17,4, 19,8 bzw. 20,1, ±0.2.

4. Die Blisterverpackung nach einem der Ansprüche 1 bis 3, wobei die Verabreichungseinheit eine Tablette ist.

5. Die Blisterverpackung nach Anspruch 4, wobei die Tablette rund oder oblong ist; und/oder wobei die Tablette planar oder bikonvex ist.

## Revendications

1. Emballage blister comprenant une unité d'administration, dans laquelle l'unité d'administration comprend un sel de fumarate de (R)-3-(6-(4-méthylphényl)-pyridin-3-yloxy)-1-aza-bicyclo[2.2.2]octane;
et dans lequel l'unité d'administration est destinée à être utilisée dans le traitement d'un trouble ou d'une maladie chez un humain, dans lequel l'unité d'administration est administrée une fois par jour, ou deux fois par jour, ou trois fois par jour.

2. Emballage blister selon la revendication 1, dans lequel le sel de fumarate de (R)-3-(6- (4-méthylphényl)-pyridin-3-yloxy)-1-aza-bicyclo[2.2.2]octane est le mono-fumarate de (R)-3-(6-(4-méthylphényl)-pyudin-3-yloxy)-1-aza-bicyclo-[2.2.2]octane.

3. Emballage de blister selon la revendication 2, dans lequel le sel de mono-fiunarate de (R)-3-(6-(4-méthylphényl)-pyridin-3-yloxy)-1-aza-bicyclo[2.2.2]octane dans le cristallin la forme est **caractérisée par** un motif XRPD avec au moins quatre des pics suivants à un angle de réfraction 2 theta (20) de 3,8, 13,7, 15,2, 17,4, 19,8 et 20,1, ± 0,2, respectivement.

4. Emballage blister selon l'une quelconque des revendications 1 à 3, dans lequel l'unité d'administration est une tablette.

5. Emballage blister selon la revendication 4, dans lequel la tablette est ronde ou oblongue; et / ou dans lequel la tablette est plane ou biconvexe.
